(19)

**Europäisches Patentamt**

**European Patent Office**

**Office européen des brevets**

(11)  **EP 4 230 290 A1**

(12)  **EUROPEAN PATENT APPLICATION**

published in accordance with Art. 153(4) EPC

(43) Date of publication:
**23.08.2023  Bulletin 2023/34**

(21) Application number: **21960357.8**

(22) Date of filing: **18.10.2021**

(51) International Patent Classification (IPC):
**B01J 23/30** (2006.01)    **B01J 23/34** (2006.01)
**B01J 35/10** (2006.01)    **C07C 2/84** (2006.01)
**C07C 9/06** (2006.01)    **C07C 11/04** (2006.01)

(52) Cooperative Patent Classification (CPC):
**Y02P 20/52**

(86) International application number:
**PCT/CN2021/124519**

(87) International publication number:
**WO 2023/060628 (20.04.2023 Gazette 2023/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.10.2021  CN 202011112296**

(71) Applicant: **Zhejiang University
Hangzhou, Zhejiang 310058 (CN)**

(72) Inventors:
• **FAN, Jie**
  **Hangzhou, Zhejiang 310058 (CN)**
• **ZHOU, Qiuyue**
  **Hangzhou, Zhejiang 310058 (CN)**
• **WANG, Junxing**
  **Hangzhou, Zhejiang 310058 (CN)**
• **ZOU, Shihui**
  **Hangzhou, Zhejiang 310058 (CN)**

(74) Representative: **Chung, Hoi Kan
Mandarin IP Limited
7 Cherry Trees
Great Shelford
Cambridge, Cambridgeshire CB22 5XA (GB)**

(54)  **COMPOSITE OXIDE CONTAINING TUNGSTATE NANOCLUSTERS, PREPARATION METHOD THEREFOR AND USE THEREOF**

(57)    The invention belongs to the field of catalysts, and particularly relates to a composite oxide containing tungstate nanoclusters, and a preparation method and application thereof. The tungstate nanocluster-containing composite oxide comprises an alkali metal element A, a tungsten element W, an auxiliary agent element M, and an oxygen element O, wherein the alkali metal element A, the tungsten element W and the auxiliary agent element M form a composite with the oxygen element O. The composite oxide as a cocatalyst can obviously improve the selectivity and the yield of $C_2$ in the oxidative coupling of methane reaction with co-fed methane and oxygen.

FIG. 5

EP 4 230 290 A1

**Description**

**Technical Field**

The invention belongs to the field of catalysts, and particularly relates to a composite oxide containing tungstate nanoclusters, and a preparation method and application thereof.

**Background**

Nanoclusters are ultra-fine particles of several to thousands of atoms, typically less than 10 nm in diameter (Journal of Molecular Catalysis A: Chemical 1999, 145, 1-44). At this scale, the species undergoes a transition from microscopic atoms/molecules to macroscopic condensed state materials, and thus nanoclusters tend to exhibit many distinct properties. Nanoclusters have been extensively studied in the past decades and their application areas include quantum dots, sensors, biomedicines, catalysis, etc. (Nanoscale, 2021, 13, 6283).

At present, most of studied nanoclusters are metal nanoclusters and oxide nanoclusters. Inorganic salt nanoclusters are rarely studied. Taking the composite oxide containing tungsten oxide nanoclusters as an example, in the prior art (such as Nature Chemistry 2009, 1, 722-728; ACS Catal. 2017, 7, 2181-2198), the method for synthesizing the tungsten oxide nanoclusters can be summarized as follows: a tungstencontaining compound (ammonium metatungstate, tungstic acid, etc.) is supported on a carrier (usually an oxide or hydroxide) by an impregnation method or a coprecipitation method, and then the tungstencontaining compound (tungstic acid or tungstate) is decomposed by high-temperature calcination to form tungsten oxide ($WO_x$). Because the carrier (such as zirconia and alumina) is in contact with $WO_x$ with strong interaction (W-O-M bond can be formed, M is non-oxygen element in the carrier, such as Zr, Al, Si and Ti), $WO_x$ can exist stably in the form of nanoclusters. Composite oxides containing tungsten oxide nanoclusters (e.g. $WO_3/ZrO_2$, $WO_3/Al_2O_3$, $WO_3/SiO_2$) are often used as solid acid catalysts. This is mainly because the surface $W^{6+}$ of $WO_x$ exists in distorted octahedral configuration ($WO_6$), and can form a Bronsted acid site-$H^{\delta+}(WO_3)_n{}^{\delta-}$ (J. Catal. 2004, 227, 479-491). As alkali metal tungstates (e.g. $Na_2WO_4$) cannot be decomposed by high-temperature annealing to form $WO_x$ and the presence of alkali metal ions can destroy the acidity of the compound, the processes used in the prior art generally do not use an alkali solution and an alkali tungstate starting material. In the limited synthesis protocols using $Na_2WO_4$ as tungsten sources (Topics in Catalysis 1998, 6, 87-99; Advances in Chemical Engineering and Science, 2014, 4, 250-257), HCl solution is also added to support tungstate ions on a carrier in the form of alkali-free tungstic acid, which contributes to decomposition to form $WO_x$. Notably, in alkali metal tungstates, such as $Na_2WO_4$, W exists as $WO_4$ configuration (J. Phys. Chem. C 2008, 112, 6869-6879), which is different from $WO_6$ coordination in $WO_x$. The configuration is quite different, and therefore the interaction between the alkali metal tungstate and the oxide support is necessarily different from that of $WO_x$ interaction with an oxide support. In this context, how to controllably synthesize alkali metal tungstate nanoclusters, particularly high concentration alkali metal tungstate nanoclusters, would be challenging. In addition, the application of alkali metal tungstate nanoclusters is also rarely reported.

Tungsten manganese catalysts are a class of classical OCM catalysts, which consist of alkali metals, tungsten, manganese and a carrier. The catalyst was originally developed in 1992 by the Shuben Li group of Lanzhou Institute of Chemical Physics (Journal of Molecular Catalysis (China) 1992, 6, 427-433). It is usually prepared by loading sodium tungstate and manganese salt on silica gel by methods such as impregnation, and then annealing at high temperature. The catalyst has drawn considerable attention due to its good OCM catalytic performance and thermal stability (ACS Catalysis 2019, 9, 5912-5928). A typical tungsten manganese catalyst composition is 1.9wt% Mn-5wt% $Na_2WO_4/SiO_2$, wherein the Mn element accounts for 1.9% of the mass of the catalyst, and $Na_2WO_4$ accounts for 5% of the mass of the catalyst. Under the experimental conditions of 800 °C, the methane space velocity of 36000mL/g/h, and $CH_4$:$O_2$:$N_2$=3:1:2.6, the methane conversion is 36.8%, and the $C_2$ hydrocarbon yield reaches 23.9%. In addition, the OCM performance of the tungsten-manganese catalyst can be optimized by means of element doping, composition regulation, and reaction condition regulation (Applied Catalysis A: General 2012, 425, 53-61).

In the preparation of tungsten-manganese catalyst, Na mainly induces $SiO_2$ changing from amorphous phase to cristobalite phase, thereby improving the dispersion of tungsten (Applied Catalysis A: General 2012, 425, 53-61; Applied Catalysis A: General 2002, 225, 271-284). The most commonly used Na source is $Na_2WO_4$. In a few catalyst preparation methods that do not use $Na_2WO_4$ as a Na source, one would not use an alkaline Na salt as a Na source, mainly because the use of an alkaline Na salt as a Na source would result in manganese elements existing as $Mn^{4+}$ form instead of active $Mn_2O_3$. This would significantly reduce the OCM catalytic activity of the tungsten manganese catalyst (Ind. Eng. Chem. Res. 2006, 45, 7077).

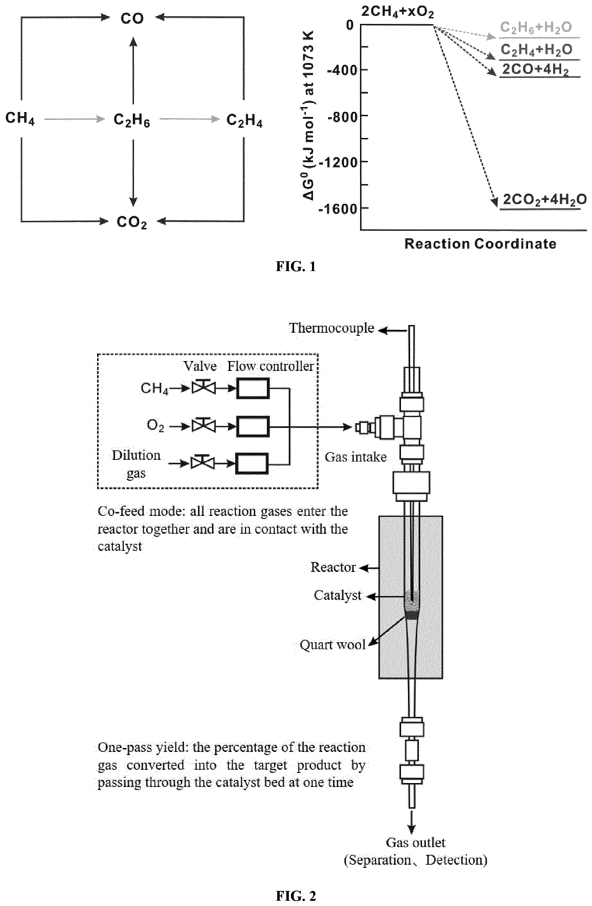

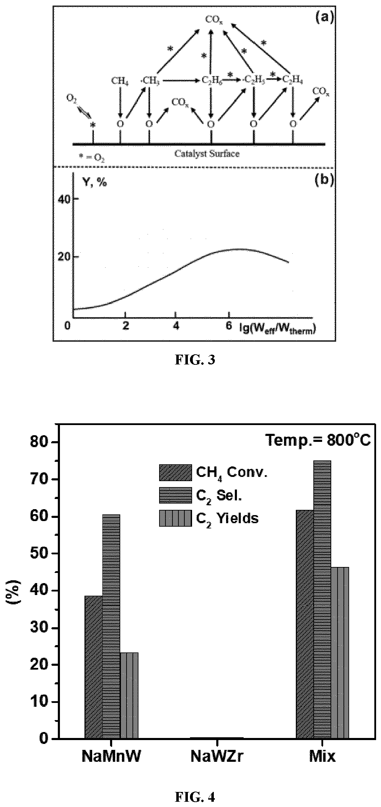

The preparation of ethane and ethylene by Oxidative Coupling of Methane (OCM) is one of the key technologies for direct utilization of methane (Energy Conversion and Management, 2019, 198, 111789; Chinese Journal of Catalysis 2021, 42, 1117-1125). Conventional research generally considers that OCM reaction follows a 'heterogeneous-homogeneous' catalytic reaction mechanism: methane is activated on the surface of a catalyst to generate methyl radicals, and then the methyl radicals are subjected to homogeneous coupling in a gas phase to generate products such as ethane and ethylene (J. Am. Chem. Soc. 1987, 109, 7900-7901; J. Mol. Catal. A: Chem. 2017, 426, 326-342). The challenge of this reaction mechanism is that the catalyst only functions in methyl radicals generating, whereas homogeneous coupling of methyl radicals in the gas phase cannot be regulated by catalyst optimization (ACS Catal. 2016, 6, 4340). Thermodynamically, methyl radical and $C_2$ species tend to react with oxygen in the gas phase to form $CO_x$, thus the $C_2$ selectivity and yield in conventional OCM catalytic systems were difficult to achieve breakthrough (Fig. 1). Arutyunov et al (J. Mol. Catal. A: Chem. 2017, 426, 326) predicted the upper limit of $C_2$ yield using kinetic simulations of gas phase reactions. They indicate that the yield of $C_2$ species '''exceed 25% only when the catalyst is able to function in the methyl radical coupling step (Figure 3). Over the past few decades, over 1000 catalysts have been developed, involving 68 elements, but the reaction mechanism has not been broken through (ChemCatChem, 2011, 3, 1935). Typically, the activation of oxygen/methane on the surface of the catalyst is regulated by means of element replacement/doping, auxiliary agent addition, catalyst morphology control, carrier regulation and the like. These methods can optimize the generation of methyl free radicals to improve the OCM reaction performance (Appl. Catal. A 2012, 425), but cannot control the conversion of methyl radicals. Theoretically, if a catalyst capable of controllably coupling methyl radicals can be developed and coupled with a catalyst for activating methane to generate methyl radicals, the limitation of the traditional OCM reaction mechanism is expected to be broken, and the breakthrough of the performance of the OCM is realized. An excellent methyl radical controlled conversion catalyst must satisfy the following requirements: 1) the catalyst has strong capacity of adsorbing methyl free radicals, and can enrich the methyl free radicals in a gas phase to the surface of the catalyst; 2) methyl free radical enriched on the surface of the catalyst can be converted into $C_2$ products with high selectivity; 3) weak oxidizing power (or difficult to activate oxygen) so that methyl free radicals and $C_2$ on the surface of the catalyst won't convert into CO and $CO_2$. As can be seen from the above requirements, the excellent methyl radical coupling catalyst itself must not have significant OCM activity because it has weak oxidizing ability and is difficult to activate methane to generate methyl radicals. Meanwhile, the catalyst has strong adsorption effect on methyl free radicals, so that the methyl free radicals are difficult to desorb into a gas phase. In the prior art, it is generally considered that methyl radicals are too reactive under OCM reaction conditions (high temperature and presence of oxygen), and it is almost impossible to achieve controlled coupling of methyl radicals on the catalyst surface (ACS Catal.2016, 6, 4340). Thus, there is no prior art to design and optimize catalysts from the point of view of methyl radical controlled surface coupling, nor has one physically mixed a promoter with no significant OCM performance in conventional OCM catalysts to promote $C_2$ selectivity and yield.

**Summary of the Invention** In order to break the limitation of the traditional methane Oxidative Coupling (OCM) reaction mechanism, the invention has carried out intensive research to develop a composite oxide containing tungstate nanoclusters, which is used as a cocatalyst for controllably coupling methyl radicals. The cocatalyst cannot be used alone as a catalyst to catalyze the OCM reaction and does not have OCM activity remarkably. But when it was physically mixed with the traditional OCM catalysts, the $C_2$ selectivity and the $C_2$ yield of the traditional OCM catalyst can be improved by more than 1.2 times (Figure 4). Therefore, the present invention has the following benefits: when the complex oxide containing tungstate nanoclusters was used as a co-catalyst in oxidative coupling of methane, and the cluster enrichment index of the complex oxide reaches the necessary density, the conventional OCM catalyst (e.g., 1.9 wt%Mn-5 wt% $Na_2WO_4/SiO_2$) can achieve a $C_2$ (ethane and ethylene) selectivity over 70%, and a $C_2$ yield over 30%.

When the tungstate nanocluster-containing composite oxide developed by the invention and the classical OCM catalyst (1.9 wt%Mn-5 wt% $Na_2WO_4/SiO_2$) were used as a catalyst composition in an OCM fix-bed reactor co-fed with methane and oxygen, the $C_2$ selectivity can reach $\geq 70\%$, and the single-pass $C_2$ yield can reach $\geq 35\%$; more preferably, the catalyst composition is capable of achieving $C_2$ selectivity $\geq 70\%$ and the single-pass $C_2$ yield $\geq 40\%$; more preferably, the catalyst composition is capable of achieving $C_2$ selectivity $\geq 70\%$ and the single-pass $C_2$ yield $\geq 45\%$.

The interpretation of the complex described therein: in the composite oxide, the valences of the alkali metal A, the tungsten element W, and the promoter element M are all positive valences, and they all achieve charge balance by combining with an oxygen element whose valences are negative valences. The compounds formed by the alkali metal A, the tungsten element W and the promoter element M with the oxygen element can be written in the form of oxides. For example, $Na_2WO_4$ can be written as $Na_2O$-$WO_3$; $ZrW_2O_8$ can be written as $ZrO_2 \cdot 2WO_3$. The detection means is as follows: the valences of the alkali metal element A, the tungsten element W, the promoter element M, and the oxygen element may be determined by X-ray photoelectron spectroscopy (XPS), and the crystal structure of the composite oxide may be determined by X-ray powder diffraction (XRD).

Wherein the atomic percentages (including a, W, and M) described herein, the atomic percentages not including oxygen, and the atomic percentages of an element in the composite oxide are defined as follows:

Atomic Percentage of an Element

$$= \frac{Total\ Atoms\ Number_{the\ element}}{Total\ Atoms\ Number_{alkali\ A} + Total\ Atoms\ Number_{tungsten\ element\ W} + Total\ Atoms\ Number_{auxiliary\ element}}$$
$$\times 100\%$$

the atomic percentage refers to the percentage ratio of all elements in the dried catalyst measured by X-ray fluorescence spectrum analysis, and the allowable test error is ±10%, preferably ±5%.

The general formula of the tungstate nanocluster is $A_xWO_y$, wherein, $0<x\leq2$ and y represents the number of oxygen atoms required to satisfy the charge balance of the formula. For example: when A is Na, x is 2, all positive charges are $2 \times 1 + 1 \times 6 = 8$ (Na is +1, W is +6, O is -2) and all negative charges are $2 \times y$. According to the charge balance, the positive charges equal to negative charges, so $2 \times y$ is 8, giving y = 4; when A is Mg, x is 1, all positive charges are $1 \times 2 + 1 \times 6 = 8$ and all negative charges are $2 \times y$. According to charge balance, the positive charges equal to negative charges, so $2 \times y$ is 8, giving y = 4.

More preferably, the particle size of the tungstate nanoclusters is 10.0 nm or less, preferably 5.0 nm or less, still more preferably 2.0 nm or less, and still more preferably 1.0 nm or less. The particle size of the tungstate nanoclusters refers to the average diameter of the nanoclusters; the detection method comprises the following steps: observing the composite oxide under a high-resolution transmission electron microscope, randomly selecting 100 tungstate nanoclusters, counting the diameters of the tungstate nanoclusters, and taking an average value. The magnification of the high-resolution transmission electron microscope is 2-30 millions, preferably 5-10 millions.

The single pass $C_2$ selectivity refers to $C_2$ (ethane and ethylene) obtained by a single pass of reactant feed gas through a catalyst bed layer, which is calculated as:

$$C_2\ selectivity = (2 \times n_{C2H6} + 2 \times n_{C2H4})/(2 \times n_{C2H6} + 2 \times n_{C2H4} + n_{CO} + n_{CO2} + 3 \times n_{C3H8} + 3 \times n_{C3H6});$$

$C_2$ Yield = $C_2$ selectivity $\times$ CH$_4$ conversion, The CH$_4$ conversion refers to the conversion efficiency of methane, and the calculation method is as follows:

$$Methane\ conversion = \left(1 - \frac{n_{CH4}}{n_{CH4} + 2 \times n_{C2H6} + 2 \times n_{C2H4} + nCO + nCO_2 + 3 \times n_{C3H8} + 3 \times n_{C3H6}}\right) \times 100\%$$

The methane conversion, $C_2$ selectivity and $C_2$ yield mentioned in the subsequent text are the results of a one-pass OCM reaction in a fix-bed reactor with co-feed of methane and oxygen, if not otherwise stated.

The invention provides a composite oxide containing tungstate nanoclusters, which comprises an alkali metal element A, a tungsten element W, an auxiliary agent element M and an oxygen element O. The alkali metal element A, the tungsten element W and the auxiliary agent element M form a compound with the oxygen element O.

The alkali metal element A is selected from any one or more of Li, Na, K, Mg, Ca, Sr and Ba.

The auxiliary agent element M is selected from any one or more of Si, Zr, Ti, Al, La, Ce and Co.

The atomic percentage of the alkali metal element A is 5 - 67%; the atomic percentage of the tungsten element W is 1 - 60%; the atomic percent of the auxiliary agent element M is 20 - 94%; the content of oxygen element in the composite oxide is the sum of the oxygen atom numbers required by the charge balance of the alkali metal element A and the tungsten element W, M;

the atomic percentage of the certain element is calculated according to the following formula,

Atomic Percentage of an Element

$$= \frac{Total\ Atoms\ Number_{the\ element}}{Total\ Atoms\ Number_{alkali\ A} + Total\ Atoms\ Number_{tungsten\ element\ W} + Total\ Atoms\ Number_{auxiliary\ element}}$$
$$\times 100\%$$

The tungstate nanocluster is composed of the alkali metal element A, the tungsten element W and the oxygen element O, and the general formula of the tungstate nanocluster is $A_xWO_y$, Wherein, $0<x\leq 2$, y represents the number of oxygen atoms required to satisfy the charge balance of the formula. The tungstate nanoclusters satisfy an enrichment index, which is defined as follows: In any region of $10 \times 10$ nm, the number of the tungstate nanoclusters is $\geq 3$; preferably, the number of the tungstate nanoclusters is $\geq 5$, more preferably $\geq 10$, and more preferably $\geq 20$;

preferably, the cluster enrichment index detection method comprises: observing the composite oxide under a high-resolution transmission electron microscope, randomly selecting 5 tungstate nanoclusters in an 10 × 10 nm area, and counting the number of tungstate nanoclusters contained in the region, and taking an average value; preferably, the magnification of the high-resolution transmission electron microscope is 2-30 millions, preferably 5-10 millions. ; preferably, the particle size of the tungstate nanocluster is ≤ 10.0 nm, preferably ≤ 5.0 nm, more preferably ≤ 2.0nm, and even more preferably ≤ 1.0 nm;

preferably, the specific surface area of the composite oxide is 0.1 to 10g/m$^2$; more preferably 0.5 to 5g/m$^2$; further preferably 1 to 2 g/m$^2$;

Preferably, three elements, namely alkali metal element A, tungsten element W and oxygen element O, in the tungstate nanocluster are uniformly distributed, wherein the uniform distribution means that any region in the tungstate nanocluster contains the alkali metal element a, the tungsten element W and the oxygen element O; preferably, the detection method for uniform distribution is as follows: observing the composite oxide under a high-resolution transmission electron microscope, performing component analysis on the tungstate nanoclusters by Energy Dispersive Spectroscopy (EDS), and randomly selecting 10 clusters, wherein any region in each cluster contains an alkali metal element A, a tungsten element W and an oxygen element O; more preferably, the magnification of the high-resolution transmission electron microscope is 20-500 thousands, preferably 50-400 thousands;

preferably, the atomic percentage of the alkali metal element a in the composite oxide is 10% to 65%, and more preferably 15% to 50%; further preferably 10 to 60% by atom, further preferably 20 to 50% by atom, further preferably 30 to 40% by atom; the atomic percentage of the tungsten element W is 2% - 55%, more preferably 5% -50%, more preferably 10% -40%, more preferably 20% -30%; the atomic percentage of the auxiliary element M is 22-92%, preferably 25-90%, preferably 30-80%, preferably 40-70%, preferably 50-60%;

preferably, the tungsten element in the tungstate nanocluster exists in the form of a tetracoordinate tungstate, wherein the tetracoordinate means that one tungsten atom has only four oxygen atoms bonded thereto; more preferably, the detection method of the four-coordinate structure is as follows: performing X-ray fine structure spectrum test on the composite oxide, and collecting $L_1$-edge and $L_3$-edge of tungsten element in the X-ray absorption near-edge structure spectroscopy (XANES) and extended X-ray absorption spectroscopy fine structure (EXAFS), by qualitative analysis and data fitting to derive coordination numbers for tungsten atoms;

preferably, after the composite oxide is calcined in the air at 800 °C for 6 hours, the particle size change value Δ1 of the tungstate nanocluster is ≤ 20%, and more preferably, Δ1 is ≤ 10%; the calculation formula is as follows:

$$\varDelta 1 = \frac{Cluster\ Size_{after\ calcination} - Cluster\ Size_{before\ calcination}}{Cluster\ Size_{before\ calcination}} * 100\%$$

the cluster enrichment index change value Δ2 of the calcined tungstate nanoclusters is ≤ 20%; more preferably, Δ2 is ≤ 10%; the calculation formula is as follows:

$$\varDelta 2 = \frac{Enrichment\ index_{after\ calcination} - Enrichment\ index_{before\ calcination}}{Enrichment\ index_{before\ calcination}} * 100\%$$

further, the alkali metal element A is at least Na, the auxiliary element M is at least Zr or Al, the composite oxide containing tungstate nanoclusters are respectively expressed as NaWZr or NaWAl, the tungstate nanoclusters are composed of alkali metal elements Na, tungsten element W and oxygen element O, and the tungstate nanoclusters have a general formula of $Na_xWO_y$, 0<x≤ 2, y represents the number of oxygen atoms required to satisfy the charge balance of the formula;

the atomic percentage of Na in the composite oxide is 5-67%; the atomic percentage of the tungsten element W is 1-60%; the atomic percent of the auxiliary agent element M is 20-94%; preferably, the atomic percentage of Na in the composite oxide is 10% to 65%, and more preferably 15% to 50%; further preferably 10 to 60% by atom, further preferably 20 to 50% by atom, further preferably 30 to 40% by atom; the atomic percentage of the tungsten element W is 2% -55%, more preferably 5% -50%, more preferably 10% -40%, more preferably 20% -30%; the atomic percentage of the auxiliary element Zr or Al is 22-92%, preferably 25-90%, preferably 30-80%, preferably 40-70%, preferably 50-60%;

in the composite oxide, 0< the molar ratio of Na: W≤5, preferably, 0.1< the molar ratio of Na: W≤4, and more preferably, 0.8≤ the molar ratio of Na: W≤3.5; 1.0≤ the molar ratio of Na: W≤3.0; further preferably, 1.5 ≤ the molar ratio of Na: W ≤ 2.0; further preferably 1.4 ≤ the molar ratio of Na: W≤1.6;

the molar ratio of W to Zr in the composite oxide is ≥ 0.1; preferably, the molar ratio of W to Zr is 0.2-100; more preferably, 0.23≤ the molar ratio of W: Zr≤10; more preferably, 0.3≤ the molar ratio of W: Zr≤1; further preferably, 0.4≤ the molar ratio of W: Zr≤0.5.

The present invention also provides a method for preparing a composite oxide containing tungstate nanoclusters, including the steps of:

1) respectively preparing a solution system 1 and a solution system 2, wherein the solution system 1 and the solution system 2 are both transparent solutions. The transparent solutions are solutions without obvious suspended matters. The solutions are not layered, and when light penetrates through the solutions, the Tyndall effect is not generated;

2) adding the solution system 1 into the stirred solution system 2 within 2-200 minutes until a turbid liquid appears, and then continuously stirring the turbid liquid for more than 1 hour, preferably more than 2 hours, and more preferably more than 3 hours; preferably, the dropping time is 2 to 200 minutes, preferably 10 to 100 minutes, and more preferably 20 to 60 minutes; preferably, the stirring is rapid, and the stirring speed is 500-1000 rpm; preferably, the stirring time is 10 minutes to 2 hours, and more preferably 30 minutes to 1 hour;

3) removing the solvent from the product obtained in the step 2) without processing, and drying the obtained solid to obtain a solid product, wherein the non-processing specifically refers to any washing, centrifuging and filtering steps;

preferably, the solvent is removed by drying the solvent, and preferably, the drying is performed by drying the product obtained in the step 2) in an atmosphere with the temperature of 30-50 °C until the solution is volatilized; preferably, the temperature is 40 °C;

preferably, the temperature at which the solid is dried is from 60 to 100 °C, more preferably from 80 to 90 °C; preferably, the drying time is 12 hours or more, more preferably 24 hours or more;

4) annealing the solid product obtained in the step 3) to obtain a composite oxide containing tungstate nanoclusters, preferably, the annealing temperature is 700-900 °C, more preferably 750-850 °C, and more preferably 800 °C; the annealing time is 3 -8 hours, preferably 4-6 hours; the ramp rate of the annealing is 2-10 °C/min, and more preferably 3-5 °C/min.

The preparation method of the solution system 1 comprises the following steps: dissolving an alkali metal element precursor of a compound raw material containing the element in a proper amount of water, and fully stirring to form a transparent solution to obtain solution system 1, wherein the pH of the transparent solution is more than 7, and more preferably ≥10;

the preparation method of the solution system 2 is selected from any one of the following methods: a) mixing and rapidly stirring a tungsten element precursor, an auxiliary agent element precursor and a proper amount of water to form a transparent solution, and obtaining a solution system 2-1; b) mixing and rapidly stirring a tungsten element precursor, an auxiliary element precursor and a proper amount of alcohol to form a transparent solution, and obtaining a solution system 2-2;

the proportion of the alkali metal element precursor, the tungsten element precursor and the auxiliary agent element precursor added into the solution system 1 and the solution system 2 accords with the following formula:

in the solution system 1 and the solution system 2, the atomic percent of a certain element is calculated according to the following formula,

Atomic Percentage of an Element

$$= \frac{Total\ Atoms\ Number_{the\ element}}{Total\ Atoms\ Number_{alkali\ A} + Total\ Atoms\ Number_{tungsten\ element\ W} + Total\ Atoms\ Number_{auxiliary\ element}}$$

$$\times 100\%$$

the atomic percentage of the alkali metal element A is 5-67%; the atomic percentage of the tungsten element W is 1-60%; the atomic percentage of the auxiliary agent element M is 20-94%;

preferably, the atomic percentage of the alkali metal element A in the composite oxide is 10-65% and more preferably 15-50%; further preferably 10-60%, further preferably 20-50%, further preferably 30-40%; the atomic percentage of the tungsten element W is 2-55%, more preferably 5-50%, more preferably 10-40%, more preferably 20-30%; the atomic percentage of the auxiliary element M is more than 22-92%, preferably 25-90%, preferably 30-80%, preferably 40-70%, preferably 50-60%.

preferably, the concentration of the alkali metal precursor in the solution system 1 is1-40 wt%, preferably 10-30 wt%, and more preferably 15-25 wt%; preferably, the concentration of the tungsten element precursor in the solution systems 2-1 and 2-2 is 1-30 wt%, preferably 5-25 wt%, and more preferably 10-20 wt%; preferably, the concentration of the precursor of the auxiliary element in the solution systems 2-1 and 2-2 is 1-50 wt%, preferably 10-40 wt%, and more preferably 20-35 wt%;

preferably, the alkali metal element precursor in the preparation method of the solution system 1 is selected from any one or more of lithium hydroxide, sodium hydroxide, lithium carbonate, sodium bicarbonate, potassium hydroxide, potassium carbonate, potassium bicarbonate, magnesium acetate, calcium hydroxide, calcium acetate, strontium hydroxide, and barium hydroxide;

preferably, the precursor of the auxiliary element in the preparation method of the solution system 2-1 is selected from any one or more of sodium silicate, zirconyl nitrate, zirconium oxychloride, zirconium di(acetate) oxide, zirconyl citrate, titanium nitrate, aluminum nitrate, lanthanum acetate, lanthanum chloride, cerium nitrate, cerium acetate, cerium chloride, cobalt nitrate, and cobalt acetate; the tungsten element precursor is selected from any one or more of sodium tungstate, cesium tungstate, tungsten ethoxide, ammonium tungsten oxide hydrate, strontium tungstate, magnesium tungstate, barium tungstate, ammonium tungstate pentahydrate, ammonium metatungstate hydrate, calcium tungstate, barium tungstate and strontium tungstate;

preferably, the precursor of the tungsten element in the preparation method of the solution system 2-2 is selected from one or two of sodium tungstate and tungsten chloride; the precursor of the auxiliary element is selected from one or more of tetraethyl orthosilicate, zirconium nitrate, zirconium n-butoxide, zirconyl nitrate, zirconium oxychloride, zirconium di(acetate) oxide, zirconium citrate, tetrabutyl titanate, aluminum sec-butoxide, aluminum isopropoxide, lanthanum nitrate, aluminum nitrate, cerium nitrate and cobalt nitrate; the alcoholic solvent is selected from one or more of methanol, ethanol, propanol, and butanol.

Further, the alkali metal element precursor is a sodium element precursor, the auxiliary element precursor is selected from a zirconium element precursor or an aluminum element precursor, the prepared composite oxide containing the tungstate nanocluster is NaWZr or NaWAl, the tungstate nanocluster is composed of an alkali metal element Na, a tungsten element W and an oxygen element O, and the general formula of the tungstate nanocluster is $Na_xWO_y$, $0<x \leq 2$, y represents the number of oxygen atoms required to satisfy the charge balance of the formula;

preferably, the alkali metal precursor in the preparation method of the solution system 1 is selected from any one or more of sodium hydroxide, sodium carbonate and sodium bicarbonate;

preferably, the precursor of the auxiliary element in the preparation method of the solution system 2-1 is selected from any one or more of zirconyl nitrate, zirconium oxychloride, zirconium di(acetate) oxide, zirconyl chloride, zirconium citrate and aluminum nitrate; the tungsten element precursor is selected from any one or more of sodium tungstate, cesium tungstate, tungsten ethoxide, ammonium tungsten oxide hydrate, strontium tungstate, magnesium tungstate, barium tungstate, ammonium tungstate pentahydrate, ammonium metatungstate hydrate, calcium tungstate, barium tungstate and strontium tungstate;

preferably, the precursor of the tungsten element in the solution system 2-2 is selected from any one or two of sodium tungstate and tungsten chloride; the precursor of the auxiliary element is selected from any one or more of zirconium nitrate, zirconium n-butoxide, zirconyl nitrate, zirconium oxychloride, zirconium di(acetate) oxide, zirconium citrate, aluminum sec-butoxide, aluminum isopropoxide and aluminum nitrate; the alcoholic solution is selected from one or more of methanol, ethanol, propanol and butanol;

preferably, 0< the molar ratio of Na: W≤ molar, preferably, 0.1 of the molar ratio of W methyl alcohol ≤4, more preferably 0.8 of the molar ratio of alcohol ≤ molar ratio; further preferably 1.0 step of the molar ratio of alcohol ≤ molar ratio; more preferably 1.5 of the molar ratio of alcohol≤ molar ratio; further preferably 1.4 step of the molar ratio of alcohol ≤ molar ratio;

The molar ratio of W:Zr is≥1; preferably, 0.2≤the molar ratio of W:Zr≤ molar ratio; further preferably 0.23≤the molar ratio of W:Zr≤ molar; further preferably 0.3 step preferably the molar ratio of Z≤ molar; further preferably 0.4 step of the molar ratio of Z≤ molar ratio;

preferably, the precursor of the auxiliary element in the preparation method of the solution system 2-2 is zirconium n-butoxide, and the precursor of the tungsten element is tungsten chloride; more preferably, the molar ratio of W:Zr≥1:9, preferably≥2:9, and more preferably ≥ 3:9; more preferably, the solution system 1 is NaOH aqueous solution, and the mass percentage of NaOH is 1-60%, preferably 10-50%, more preferably 15-40%;

preferably, the precursor of the auxiliary element in the preparation method of the solution system 2-2 is aluminum isopropoxide, the precursor of the tungsten element is tungsten chloride, and the molar ratio of the tungsten element to the aluminum element is≥1:9, preferably≥2:9, and more preferably≥3:9; more preferably, the solution system 1 is NaOH aqueous solution, and the mass percentage of NaOH is 1-60%, preferably 10-50%, more preferably 15-40%;

preferably, the precursor of the auxiliary element in the preparation method of the solution system 2-2 is zirconium n-butoxide, the precursor of the tungsten element is sodium tungstate, and the molar ratio of W:Zr≥1:9, preferably≥2:9, and more preferably≥3:9; more preferably, the solution system 1 is KOH with a mass percentage of 1-60%, preferably 10-50%, and more preferably 15-40%;

preferably, the precursor of the auxiliary element in the preparation method of the solution system 2-2 is aluminum isopropoxide, the precursor of the tungsten element is sodium tungstate, and the molar ratio of W:Al≥1:9, preferably≥2:9, and more preferably≥3:9; more preferably, the solution system 1 is KOH with a mass percentage of 1-60%, preferably 10-50%, and more preferably 15-40%.

The invention also provides a cocatalyst, which comprises the composite oxide containing tungstate nanoclusters;

preferably, the catalyst promoter cannot be used alone as a catalyst for Oxidative Coupling of Methane (OCM) reaction; preferably, the cocatalyst does not have significant OCM activity; preferably, the detection method for the unsignificant OCM activity is as follows: in a bed reactor of co-feeding methane and oxygen, when the catalyst is used as a OCM catalyst and the gas-space-time velocity is≥20000mL/g/h, the bed temperature of a catalyst is≤800 °C, and the reaction pressure is 1atm, the single-pass $C_2$ yield is not higher than 5%; preferably, the yield is quantitatively determined by gas chromatography.

The present invention also provides a catalyst composition comprising the composite oxide of claim and at least one $OCM_{catalyst}$ having OCM activity;

Preferably, the $OCM_{catalyst}$ having OCM activity means that the catalyst can be used alone for catalyzing OCM reaction; preferably, the meaning of OCM activity is: in a bed reactor of co-feeding methane and oxygen, when the gas-space-time velocity of the catalyst is≥20000mL/g/h, the bed temperature of the catalyst is≤800 °C, and the reaction pressure is 1 atm, the catalyst is used as an OCM catalyst and have a single pass $C_2$ yield≥5%;

preferably, the $OCM_{catalyst}$ has significant OCM activity, and the meaning thereof is as follows: in a bed reactor co-fed with methane and oxygen, when the catalyst is used as an OCM catalyst, the gas-space-time velocity is ≥ 20000mL/g/h, the catalyst bed temperature is≤800 °C, and the reaction pressure is 1 atm, the catalyst is used as an OCM catalyst and haa a single pass $C_2$ yield≥15%;

preferably, the mass ratio of the $OCM_{catalyst}$ to the composite oxide is (0.1-50.0):1.0; more preferably, the mass ratio is (0.5-20.0):1.0; more preferably, the mass ratio is (1.0-10.0):1.0; more preferably, the mass is (2.0-4.0):1.0; preferably, the yield is quantitatively determined by gas chromatography;

preferably, the $OCM_{catalyst}$ is selected from one or more of tungsten manganese catalyst, rare earth metal oxide, perovskite compound, alkali metal and alkaline earth metal oxide and derivatives of the above catalysts; more preferably tungsten manganese catalyst, lanthanum oxide, samarium oxide, Li/MgO, $Ca/CeO_2$; Preferably, the tungsten manganese catalyst is $Mn-Na_2WO_4/SiO_2$;

Preferably, the distance between the the $OCM_{catalyst}$ and the composite oxide is≤3 mm;

Preferably, the catalyst composition is prepared by mixing the composite oxide and $OCM_{catalyst}$ in a physical mixing mode;

preferably, the $OCM_{catalyst}$ is a compound containing a second alkali metal element, a tungsten element, a manganese element, an oxygen element, and a fifth component element; more preferably, the fifth component element is selected from any one or more of Al, Si, Ti, Zr, C, and N, even more preferably, the fifth component element is selected from any one or more of Al, Si, and Ti, and more preferably, the fifth component element is selected from Si; more preferably, the second alkali metal is selected from any one or more of Li, Na and K; more preferably, the second alkali metal is selected from Na;

preferably, the mass percent of the second alkali metal element accounts for the $OCM_{catalyst}$ is 0.1-2.0 wt%; more preferably, the mass percent of the second alkali metal element for the $OCM_{catalyst}$ is 0.5-1.5 wt%;

preferably, the tungsten element accounts for the $OCM_{catalyst}$ is 0.1-5.0 wt%, preferably, the mass percent of the tungsten element accounts for the $OCM_{catalyst}$ is 2.0-4.0 wt%;

preferably, the manganese element accounts for the $OCM_{catalyst}$ is 0.1-10.0 wt%, preferably, the mass percent of the manganese element accounts for the $OCM_{catalyst}$ is 1.0-4.0 wt%.

Preferably, the catalyst further comprises an additive; more preferably, the additive is selected from any one or more of heat transfer agent, mass transfer aggent, forming aggent, wear-resistant enhancing agents, dispersing agents, stabilizing agents and the like.

Preferably, the catalyst composition catalyzes the OCM reaction under the same reaction conditions with selectivity and yield higher than those of the $OCM_{catalyst}$ alone by more than 1.2 times;

preferably, the catalyst composition is capable of realizing $C_2$ selectivity ≥ selectivity and the single-pass $C_2$ yield ≥ pass yield in an OCM bed reactor with co-feeding of methane and oxygen; more preferably, the catalyst composition is capable of achieving $C_2$ selectivity ≥ selectivity and the single-pass $C_2$ yield ≥ 40% in an OCM bed reactor co-fed with methane and oxygen; more preferably, the catalyst composition is capable of achieving $C_2$ selectivity $\geq$ selectivity and the single-pass $C_2$ yield $\geq$ 45% in an OCM bed reactor co-fed with methane and oxygen.

Further, the $OCM_{catalyst}$ is $Mn/Na_2WO_4/SiO_2$, and the composite oxide is NaWZr; preferably, the mass ratio of the $OCM_{catalyst}$ to the composite oxide is 4:1-0.5:1; more preferably 2:1-1:1; preferably, the catalyst composition can realize C2 selectivity $\geq$ 70% and single-pass $C_2$ yield $\geq$ 35% in the OCM bed reactor cofed with methane and oxygen; more preferably, the catalyst composition can realize $C_2$ selectivity$\geq$ selectivity and single-pass $C_2$ yield$\geq$40% in the OCM bed reactor cofed with methane and oxygen; more preferably, the catalyst composition can realize $C_2$ selectivity$\geq$ selectivity and single-pass $C_2$ yield$\geq$45% in the OCM bed reactor cofed with methane and oxygen; preferably, in an OCM bed reactor with co-feeding of methane and oxygen, the $C_2$ selectivity of the catalyst composition $Mn/Na_2WO_4/SiO_2$-NaWZr can be improved to 1.3 times of $Mn/Na_2WO_4/SiO_2$, the single pass $C_2$ yield is 1.2 times of $Mn/Na_2WO_4/SiO_2$.

Further, the $OCM_{catalyst}$ is $La_2O_3$ and the composite oxide NaWZr is the composite oxide of Na, W and Zr, and the mass ratio of the $OCM_{catalyst}$ to the composite oxide is 4:1-1:1; more preferably from 2:1-1:1; preferably, in an OCM bed reactor with co-feeding of methane and oxygen, the $C_2$ selectivity of the catalyst composition $La_2O_3$-NaWZr can be improved to 1.3 times of $La_2O_3$, the single pass $C_2$ yield is 1.2 times of $La_2O_3$. Further, the $OCM_{catalyst}$ is $Sm_2O_3$ and the composite oxide NaWZr is the composite oxide of Na, W and Zr, and the mass ratio of the $OCM_{catalyst}$ to the composite oxide is 4:1-1:1; more preferably from 2:1-1:1;

preferably, in an OCM bed reactor with co-feeding of methane and oxygen, the $C_2$ selectivity of the catalyst composition $Sm_2O_3$-NaWZr can be improved to 2.0 times of $Sm_2O_3$, the single pass $C_2$ yield is 1.8 times of $Sm_2O_3$. Further, the $OCM_{catalyst}$ is Li/MgO and the composite oxide NaWZr is the composite oxide of Na, W and Zr, and the mass ratio of the $OCM_{catalyst}$ to the composite oxide is 4:1-1:1; more preferably from 2:1-1:1; preferably, in an OCM bed reactor with co-feeding of methane and oxygen, The $C_2$ selectivity of the catalyst composition Li/MgO-NaWZr can be improved to 2.0 times of Li/MgO. The single pass $C_2$ yield is 1.45 times of Li/MgO. Further, the $OCM_{catalyst}$ is $Ca/CeO_2$ and the composite oxide NaWZr is the composite oxide of Na, W and Zr, and the mass ratio of the $OCM_{catalyst}$ to the composite oxide is 4:1-1:1; more preferably from 2:1-1:1; preferably, in an OCM bed reactor with co-feeding of methane and oxygen, the $C_2$ selectivity of the catalyst composition $Ca/CeO_2$-NaWZr can be improved to 1.3 times of $Ca/CeO_2$, the single pass $C_2$ yield is 1.5 times of $Ca/CeO_2$. Further, the catalyst composition is $Mn-Na_2WO_4/SiO_2$-NaWAl and the $OCM_{catalyst}$ is $Mn-Na_2WO_4/SiO_2$ and the composite oxide NaWZr is the composite oxide of Na, W and Zr. The mass ratio of the $OCM_{catalyst}$ to the composite oxide is 4:1-1:1; more preferably from 2:1-1:1; preferably, the catalyst composition $Mn-Na_2WO_4/SiO_2$-NaWAl can realize $C_2$ selectivity $\geq$ 70% and single-pass recovery $C_2$ yield $\geq$ 35% in the OCM bed reactor cofed with methane and oxygen; more preferably, the catalyst composition can realize $C_2$ selectivity$\geq$ selectivity and single-pass $C_2$ yield$\geq$40% in the OCM bed reactor cofed with methane and oxygen; more preferably, the catalyst composition can realize $C_2$ selectivity$\geq$ selectivity and single-pass $C_2$ yield$\geq$45% in the OCM bed reactor cofed with methane and oxygen; preferably, the $C_2$ selectivity of $Mn-Na_2WO_4/SiO_2$-NaWAl can be improved to 1.3 times of $Mn-Na_2WO_4/SiO_2$, and the single pass $C_2$ yield is 1.2 times of $Mn/Na_2WO_4/SiO_2$.

Further, the $OCM_{catalyst}$ is $La_2O_3$, the composite oxide is NaWAl which is a composite oxide of Na, W and Al, and the mass ratio of the $OCM_{catalyst}$ to the composite oxide is 4:1-1:1; more preferably from 2:1 to 1:1;

preferably, the catalyst composition $La_2O_3$-NaWAl in an OCM bed reactor co-feeding with methane and oxygen, the $C_2$ selectivity of $La_2O_3$-NaWAl can be improved to 1.3 times of $La_2O_3$, and the single pass $C_2$ yield is 1.2 times of $La_2O_3$. Further, the $OCM_{catalyst}$ is $Sm_2O_3$, the composite oxide is NaWAl which is a composite oxide of Na, W and Al, and the mass ratio of the $OCM_{catalyst}$ to the composite oxide is 4:1-1:1; more preferably from 2:1 to 1:1; preferably, the catalyst composition $Sm_2O_3$-NaWAl in an OCM bed reactor co-feeding with methane and oxygen, the $C_2$ selectivity of $Sim_2O_3$-NaWAl can be improved to 1.2 times of $Sm_2O_3$, and the single pass $C_2$ yield is 1.5 times of $Sm_2O_3$.

Further, the $OCM_{catalyst}$ is Li/MgO, the composite oxide is NaWAl which is a composite oxide of Na, W and Al, and the mass ratio of the $OCM_{catalyst}$ to the composite oxide is 4:1-1:1; more preferably from 2:1 to 1:1; preferably, the catalyst composition Li/MgO-NaWAl is in an OCM bed reactor with co-feeding of methane and oxygen, and the $C_2$ selectivity of the Li/MgO-NaWAl can be increased to 1.7 times of Li/MgO, and the single-pass $C_2$ yield is 1.8 times of that of Li/MgO.

Further, the $OCM_{catalyst}$ is $Ca/CeO_2$, the composite oxide is NaWAl which is a composite oxide of Na, W and Al, and the mass ratio of the $OCM_{catalyst}$ to the composite oxide is 4:1-1:1; more preferably from 2:1 to 1:1; preferably, the catalyst composition $Ca/CeO_2$-NaWAl in an OCM bed reactor co-feeding with methane and oxygen, the $C_2$ selectivity of of NaWAl can be improved to 1.3 times of $Ca/CeO_2$, and the single-pass $C_2$ yield is 1.6 times of $Ca/CeO_2$.

The invention also provides the use of the catalyst composition in chemical reactions; preferably, the chemical reaction is a radical conversion reaction; more preferably, the chemical reaction is oxidative coupling of methane. The oxidative coupling of methane refers to a process that carbon-hydrogen bonds of methane are broken under the action of a catalyst, the separated hydrogen and oxygen react to generate water, and carbon-carbon bonds are formed to prepare $C_{2+}$ hydrocarbon.

Further, the oxidative coupling of methane takes methane and oxygen as raw material gases, and the reaction is carried out on a bed reactor, and the product comprises $C_2$ hydrocarbons, hydrocarbons and $C_3$ hydrocarbon;

preferably, the feed gas further comprises a diluent gas; more preferably, the diluent gas is selected from at least one of nitrogen, helium, argon;
the ratio of methane to oxygen is 1.0-20.0, preferably 2.0-6.0, more preferably 2.0-5.0;
preferably, the pressure of the raw material gas is 0.1-20.0 MPa, preferably 1.0-8.0 MPa, and more preferably 2.0-6.0 MPa;
preferably, the temperature of the reaction is 600-900 °C, preferably 700-850 °C;
preferably, the space velocity of the reaction is 500-50000 $h^{-1}$, preferably 1000-35000 $h^{-1}$.

The effect of the present invention is that the composite oxide containing tungstate nanoclusters can be used as a cocatalyst with traditional OCM catalyst to make the $C_2$ selectivity of oxidative coupling of methane reaching more than 70%, and the $C_2$ yield reaching more than 30%. In particular, because the researchers usually optimize the synthesis of traditional tungsten-manganese OCM catalysts from the viewpoint of improving OCM performance, they have no motivation to use basic sodium salts ($Na_2CO_3$, $Na_2SiO_3$) or NaOH and other basic solutions (pH>7) to prepare tungsten-manganese catalysts. In this context, most of tungsten-manganese catalysts are synthesized by impregnation using $Na_2WO_4$ and manganese salts as precursors, and almost no researchers use the alkaline solution of Na to prepare tungsten-manganese catalysts by co-precipitation. In the research of the present inventors, it was unexpectedly found that in the sodium tungstate-containing catalyst prepared by co-precipitation method using an alkaline solution of Na such as NaOH, sodium tungstate exists in the form of nanoclusters, and the sodium tungstate clusters meet the cluster enrichment index (refers to any region of $10 \times 10$ nm$^2$ comprising the tungstate nanocluster, the number of tungstate nanoclusters $\geq 3$). The catalyst containing the sodium tungstate nanoclusters itself does not have OCM activity (when the catalyst is used as an OCM catalyst in a bed reactor with co-feeding of methane and oxygen and has the gas-space-time velocity $\geq 20000$ mL/g/h, the catalyst bed temperature $\leq 800$ °C, and the reaction pressure of 1 atmosphere, the single-pass $C_2$ yield is not higher than 5%), but the catalyst can be used as a cocatalyst to improve the $C_2$ selectivity and yield of the traditional OCM catalyst. This prompted the present inventors to further study composite oxides containing tungstate nanoclusters of different compositions as promoters, and further completed the present invention. The research of the invention is obviously different from the research of the traditional tungsten-manganese catalyst, and no other literature has reported similar research at present.

Brief Description of the Invention

FIG. 1 shows the reaction pathway of OCM along with the corresponding graphical representation of Gibbs free energy.
FIG. 2 shows a schematic diagram of a co-feed bed reactor for methane and oxygen.
FIG. 3(a) shows a network diagram of the OCM reaction (reference: Beck, B. et al., Catal. Today 2014, 228, 212).
FIG. 3(b) shows the correlation between the yield of $C_2$ products and the rate of methyl radical generation obtained through kinetic simulations (reference: Arutyunov, V. et al., J. Mol. Catal. A: Chem. 2017, 426, 326).
FIG. 4 shows the experimental results of the NaMnW-NaWZr system, illustrating the outcomes of Example 1.

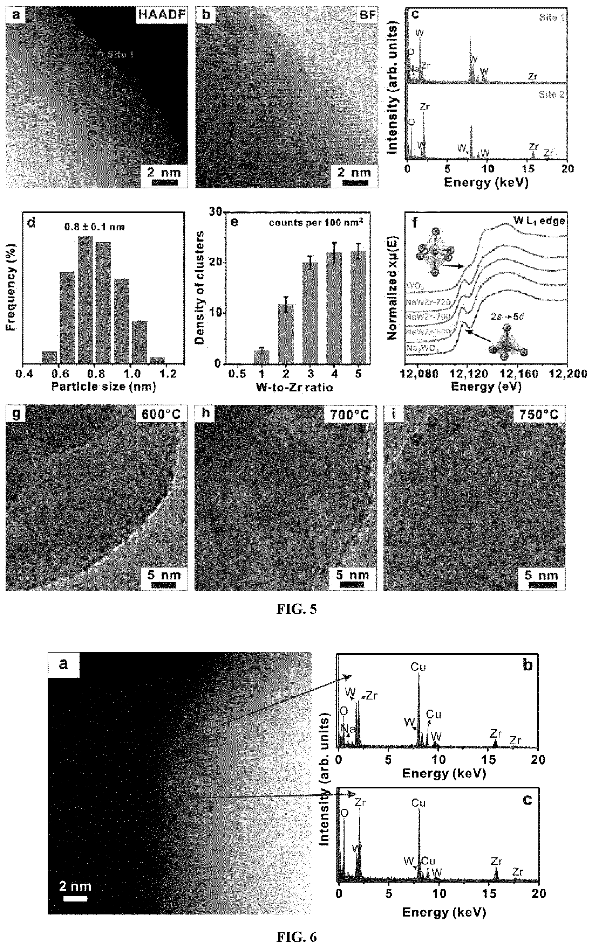

FIG. 5 shows structural characterization of NaWZr. In particular, (a, b) representative high-angle annular dark-field (HAADF) and bright-field (BF) scanning transmission electron microscopy (STEM) images of NaWZr are shown. Unless otherwise specified, the molar ratio of tungsten to zirconium is 3:9. (c) EDS point spectra of the region indicated by the red circle in Figure 1(a). The Cu signal originates from the Cu grid. (d) Size distribution of NaxWOy clusters in NaWZr. (e) Coarse statistics of NaxWOy cluster density in NaWZr with different W-Zr ratios. (f) W L3-edge X-ray absorption near-edge structure (XANES) spectra of NaWZr, Na2WO4, and WO3.
FIG. 6 shows the energy-dispersive X-ray spectroscopy (EDS) analysis of Example 1.
FIG. 7 shows the high-angle annular dark-field scanning transmission electron microscopy (HAADF-STEM) image of Example 1. Some NaxWOy clusters are highlighted with green rectangular markers.

FIG. 8 shows the EDS mapping image of Example 1.

FIG. 9 shows the Raman spectrum of Implementation 1, compared with commercially available ZrO2. The characteristic Raman band around 925 cm-1 can be attributed to the W-O-Zr bond.

FIG. 10 shows the Zr K-edge X-ray absorption near-edge structure (XANES) spectrum of Implementation 1. Compared to ZrO2, the Zr K-edge of NaWZr shows a noticeable blue shift, indicating the charge transfer from Zr to W through the Zr-O-W bond.

FIG. 11 shows representative bright-field (BF) and high-angle annular dark-field (HAADF) scanning transmission electron microscopy (STEM) images of NaWZr with a W:Zr molar ratio of 1:9. In the images, individual dispersed and aggregated tungsten species are marked with red circles, while NaxWOy clusters are highlighted with green squares.

FIG. 12 shows representative high-angle annular dark-field scanning transmission electron microscopy (HAADF-STEM) images of NaWZr with a W:Zr molar ratio of 2:9. In the images, individual dispersed and aggregated tungsten species are marked with red circles, while NaxWOy clusters are highlighted with green squares.

FIG. 13 shows representative bright-field (BF) and high-angle annular dark-field (HAADF) scanning transmission electron microscopy (STEM) images of NaWZr with a W:Zr molar ratio of 4:9. In the images, some NaxWOy clusters are marked with green squares.

FIG. 14 shows representative bright-field (BF) and high-angle annular dark-field (HAADF) scanning transmission electron microscopy (STEM) images of NaWZr with a W:Zr molar ratio of 5:9. In the images, some NaxWOy clusters are marked with green squares.

FIG. 15 shows typical bright-field (BF) and high-angle annular dark-field (HAADF) scanning transmission electron microscopy (STEM) images of NaWZr with a W:Zr molar ratio of 0.5:9. In the images, dispersed tungsten species are marked with red circles.

FIG. 16 shows a typical high-angle annular dark-field scanning transmission electron microscopy (HAADF-STEM) image of the NaWAl catalyst.

FIG. 17 shows a STEM spectrum of Na2WO4 in the form of large particles.

FIG. 18 shows the impact of the distance between the OCM catalyst and the composite oxide on the catalytic performance of OCM.

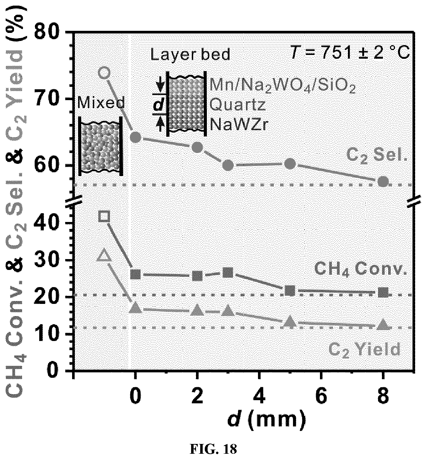

From FIG. 18, it can be observed that the distance between the OCM catalyst and the composite oxide significantly impacts the catalytic performance of OCM. The OCM catalyst exhibits superior performance when the distance between the catalyst and the composite oxide is ≤3mm. Furthermore, the catalytic effect becomes increasingly favorable as the distance decreases.

**Detailed Description of the Invention**

The following description of the embodiments is provided for a better understanding of the invention and should not be taken as limiting the invention.

Wherein the following examples are described using the relevant methods:

1. The Mn/Na$_2$WO$_4$/SiO$_2$ catalysts are commercially available or can be synthesized by wet impregnation methods, as described in the literature (Journal of Molecular Catalysis (China) 1992, 6, 427-433). The specific synthesis steps are as follows: typically, an aqueous manganese (II) nitrate solution (50 wt%) and sodium tungstate hydrate were dissolved in an appropriate amount of deionized water. A commercially available SiO$_2$ carrier with a surface area of ~200 m$^2$/g is added to the above solution with continuous agitation and then dried overnight at 105°C. The ratio of manganese nitrate and sodium tungstate satisfies that the mass percentage of manganese (Mn) element to Mn/Na$_2$WO$_4$/SiO$_2$ catalyst is 2 wt%, and sodium tungstate (Na$_2$WO$_4$) is 5 wt% of Mn/Na$_2$WO$_4$/SiO$_2$ catalyst. Thereafter, the dried solid was calcined at 800 °C for 4 hours to obtain the final catalyst.

2. In the following examples, the composition of the samples was measured using an XRF-1800 X-ray fluorescence spectrometer from Shimadzu corporation, Japan; the specific surface area of the sample is determined by N$_2$ absorption and desorption method using an MICROMERICS ASAP2020 (USA), and the measured specific surface area refers to BET specific surface area; High angle annular dark field scanning transmission electron microscopy (HAADF-STEM) images and elemental mapping were acquired ex-situ on a Cs-corrected Titan Chemi-STEM operating at an accelerating voltage of 200 kV. The Raman spectrum of the catalyst was measured at room temperature with visible laser excitation (532 nm) on a Horiba-Jobin-Yvon. In situ X-ray absorption fine structure (XAFS) spectra were measured on beamline 14W at the Shanghai Synchrotron Radiation Facility (SSRF) in China, using a Si(111) double crystal monochromator and an in situ cell (HTC-MSXAS-500, Beijing Scistar Technology Co., Ltd., maximum working temperature: 1000 K).

3. The OCM tests were performed in a quartz fix-bed reactor tube with an inner diameter of 8 mm under atmospheric pressure. The catalyst bed was placed on a quartz wool plug in the constant-temperature zone of the furnace. A thermocouple well with an outer diameter of 6 mm was inserted into the quartz reactor to monitor the bed temperature and to further reduce the free space volume. $CH_4$, $O_2$, and $N_2$ in a ratio of 3:1:2.7 were cofed into the reactor through mass flow controllers. The total flow rate was 67 mL min$^{-1}$. The products were analyzed by an online gas chromatograph equipped with an FID detector and a TCD detector. Molecular sieve 5A column was coupled with Porapak N column to separate $CO_2$, $CH_4$, $O_2$ and $N_2$ while aluminum trioxide capillary column was used to separate hydrocarbon products ($CH_4$, $C_2H_4$, $C_2H_6$, $C_3H_6$ and $C_3H_8$). All the carbon-containing products except $C_3$ species were quantified by TCD. The trace $C_3$ species were quantified by FID. In order to eliminate the error associated with the different detection methods, FID signals were calibrated to make sure the quantification of methane, ethane, and ethylene by FID was exactly the same as that quantified by TCD. All the catalysts were calcined in air at 800 °C for 5 h before catalytic test. For testing $OCM_{catalyst}$ (e.g., $Mn/Na_2WO_4/SiO_2$, $La_2O_3$ etc.) and cocatalyst (e.g., NaWZr) alone, 200 and 100 mg of catalyst was loaded, specifically. When testing the catalyst composition formed by $OCM_{catalyst}$ and cocatalyst, unless otherwise specified, the dosage of $OCM_{catalyst}$ is 200 mg, and the dosage of cocatalyst is 100 mg.

The $CH_4$ conversion and product selectivity were calculated based on a carbon atom basis of the inlet and outlet gases. The outlet gases were corrected for gas expansion by using $N_2$ as an internal standard.

$$CH_4\ Conv. = \left(1 - \frac{nCH_{4\,outlet}}{nCH_{4\,outlet} + \sum x \times n[products]_{outlet}}\right) \times 100\% \qquad (3)$$

where $x$ is the number of carbon atom in the products.

The products selectivity was calculated on a carbon atom basis of the outlet products (i.e., $C_2H_4$, $C_2H_6$, CO, $CO_2$, $C_3H_6$ and $C_3H_8$). $C_2$ products include both $C_2H_4$ and $C_2H_6$.

$$C_2\ Sel. = \frac{2 \times nC_2H_4 + 2 \times nC_2H_6}{2 \times nC_2H_4 + 2 \times nC_2H_6 + 1 \times nCO + 1 \times nCO_2 + 3 \times nC_3H_6 + 3 \times nC_3H_8} \times 100\% \quad (4)$$

$$CO\ Sel. = \frac{1 \times nCO}{2 \times nC_2H_4 + 2 \times nC_2H_6 + 1 \times nCO + 1 \times nCO_2 + 3 \times nC_3H_6 + 3 \times nC_3H_8} \times 100\% \quad (5)$$

$$CO_2\ Sel. = \frac{1 \times nCO_2}{2 \times nC_2H_4 + 2 \times nC_2H_6 + 1 \times nCO + 1 \times nCO_2 + 3 \times nC_3H_6 + 3 \times nC_3H_8} \times 100\% \quad (6)$$

$$C_2\ Yield = CH_4\ Conv. * C_2\ Sel. * 100\% \qquad (7)$$

The carbon balance was calculated according to:

$$Carbon\ balance = \frac{nCH_{4\,outlet} + \sum x \times n[products]_{outlet}}{nCH_{4\,inlet}} \times 100\% \qquad (8)$$

where $x$ is the number of carbon atom in the products. Generally, the carbon balance was higher than 95%.

**Example 1:**

Weighing a certain amount of NaOH and dissolving the NaOH in water to obtain a clear NaOH aqueous solution 1 with the mass concentration of 23 wt%; 3.453 g of zirconium n-butoxide (80 wt%) and 1.188 g of tungsten hexachloride are weighed and added into 30 mL of ethanol, and the mixture is fully stirred and dissolved to obtain a clear solution 2; adding 2 mL of NaOH aqueous solution 1 into the solution 2 within 10 minutes under rapid stirring (the stirring speed is 800 rpm) and continuously stirring for 3 hours to obtain a turbid solution. The obtained turbid solution was dried at 40 °C and aged in an 80 °C oven for 12 hours. The obtained solid was calcined at 800 °C in the air for 5 hours to obtain the catalyst. The atomic ratio of W atoms to zirconium atoms in the catalyst was 3:9.

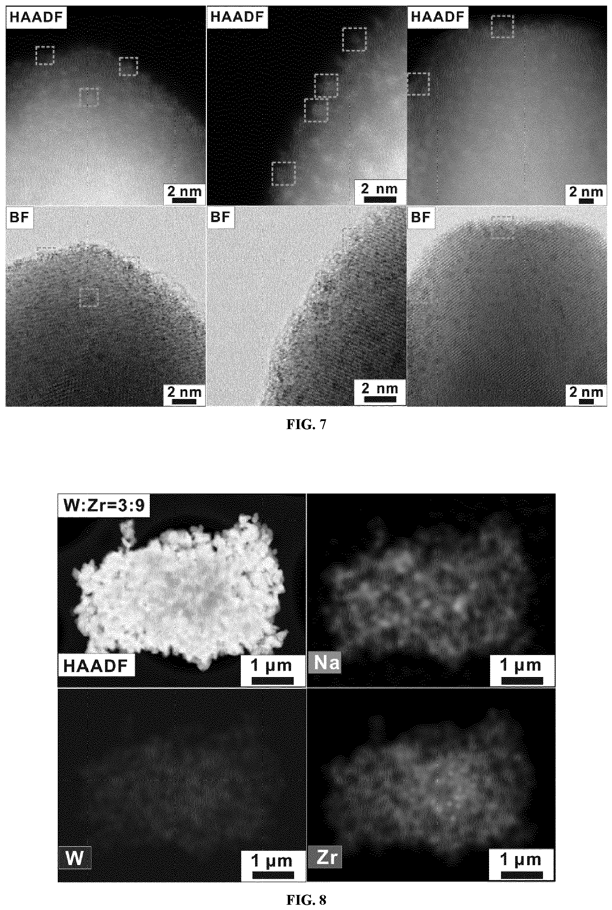

The high-resolution transmission electron microscope images of the catalyst are shown in figure 5a,5b, and 7, and the catalyst is obviously rich in nanoclusters. The white clusters and the dark non-cluster regions in the graph were subjected to element scanning analysis (figure 5c and 6), respectively, and it was found that the white clusters were $Na_xWO_y$ clusters rich in Na, W and O elements, while the non-cluster regions were mainly $ZrO_2$ formed from the auxiliary element Zr. The average particle size of the $Na_xWO_y$ nanoclusters in this catalyst was found to be 0.8 nm (as shown in d in figure 5). Further counting the number of $Na_xWO_y$ nanoclusters in the 10*10 $nm^2$ region, it was found that the number was 21 (e in Figure 5).

EDS Mapping of the catalyst is shown in Fig. 8, and Na, W and Zr elements are uniformly distributed in the catalyst.

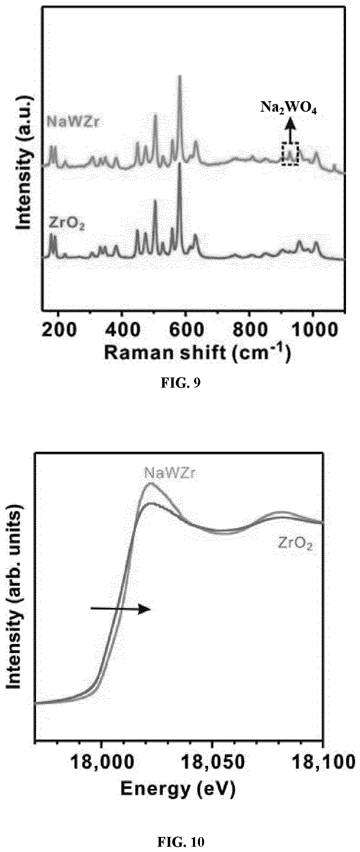

W $L_1$ edge XANES spectra of NaWZr shown in Fig. 5f exhibit characteristic pre-edge peaks at ~ 12117 eV, which is similar to that of $Na_2WO_4$ and different from $WO_3$. These results suggest that W species in the catalyst feature tetrahedral $WO_4$ (i.e., tungstate) rather than octahedral $WO_6$ (i.e., tungsten oxide) structures. The Raman spectra of the catalyst was shown in Fig. 9. Comparing to $ZrO_2$, the catalyst exhibits a characteristic peak at 925$cm^{-1}$, which could be attributed to $Na_2WO_4$. In addition, the Zr K-edge XANES spectrum of the catalyst is shown in Fig. 10. Compared with $ZrO_2$, the Zr K-edge of NaWZr is significantly blue shifted, indicating that Zr transfers charge to W. The existence of strong interaction between Zr and W is the key to achieve the high enrichment index of $Na_xWO_y$ nanoclusters. On the one hand, it can stabilize $Na_xWO_y$ nanoclusters and allow them to exist in the form of nanoclusters. On the other hand, the sintering agglomeration of adjacent $Na_xWO_y$ nanoclusters is avoided, so that multiple $Na_xWO_y$ nanoclusters can exist in the region of 10* 10 $nm^2$.

**Examples 2-16** were synthesized according to the method described in example 1, with slightly different parameters from example 1, and the specific synthesis parameters and structural parameters are shown in (table 1-1 - 1- 4).

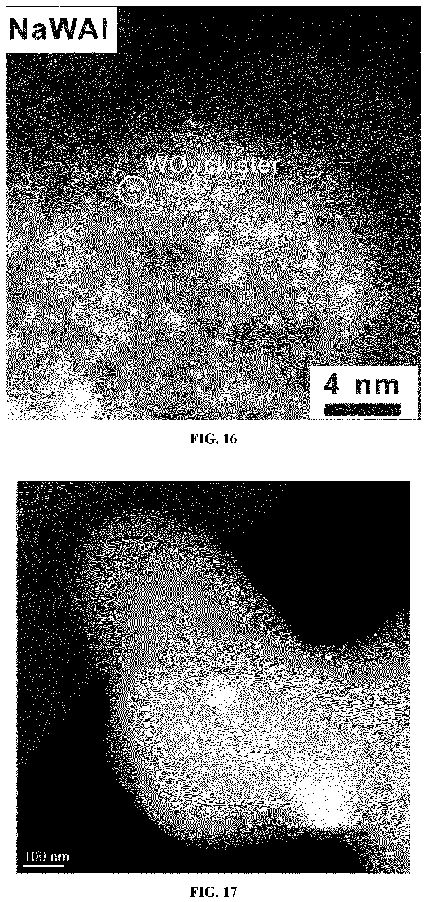

Example 2 differs from example 1 in that the auxiliary element used in example 2 is Al. The high-resolution transmission electron microscopy image of the catalyst is shown in Figure 16, from which it can be clearly seen that the catalyst is rich in nanoclusters. It was found that the average particle size of $Na_xWO_y$ nanoclusters in the catalyst was 0.9 nm. Further statistics of the number of $Na_xWO_y$ nanoclusters in the 10 × 10 $nm^2$ region showed that the number was 21.

By analyzing the structural parameters (Table 1-1) of examples 1 to 7, it was concluded that when the alkali metal element was Na, K, or Li and the auxiliary element was Zr or Al, composite oxides containing tungstate nanoclusters can be obtained. The nanoclusters have an average particle size of 0.8-0.9 nm, the number of $Na_xWO_y$ nanoclusters is ~21 in the 10×10$nm^2$ region.

By analyzing examples 1 and 8, the compound oxide containing tungstate nanoclusters can be obtained if the tungsten source is chloride or sodium tungstate, and the nanoclusters have an average particle size of 0.8-0.9 nm, the number of $Na_xWO_y$ nanoclusters is ~21 in the 10×10$nm^2$ region.

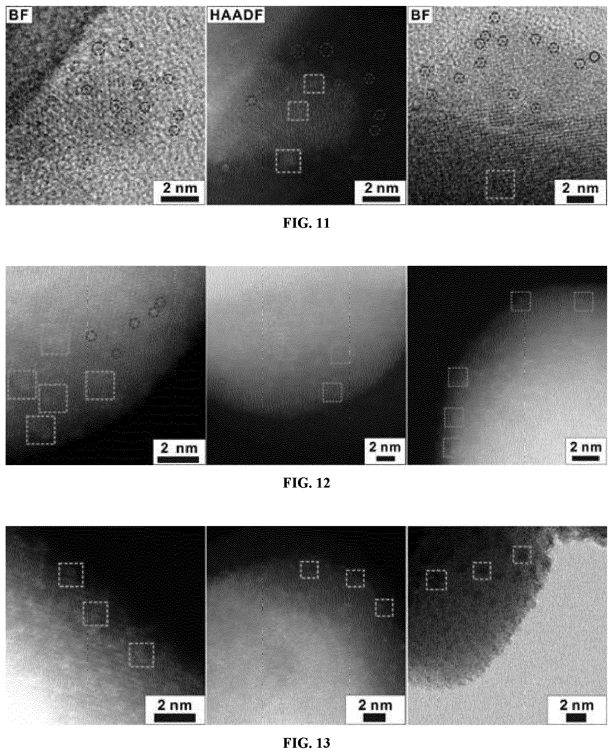

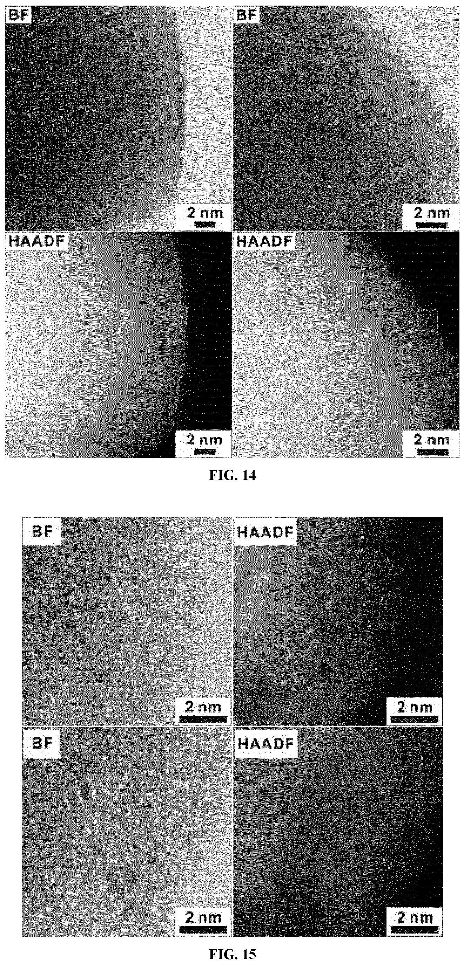

STEM images of Examples 9-13 are shown in Fig. 11-15, respectively. Examples 9 to 13 differ from example 1 in the molar ratio of W to Zr. As can be seen from Fig. 11 to 15, composite oxides containing tungstate nanoclusters were obtained when the molar ratio of W to Zr was 1:9.2:9,3:9,4:9,5:9, and 0.5:9. However, the molar ratio of W to Zr influences $Na_xWO_y$ enrichment index of nanoclusters. The number of tungstate nanoclusters in the 10×10$nm^2$ region in Examples 1 and 9-13 are summarized in Figure 5e. Clearly, the high feeding molar ratio of W:Zr is critical for the formation of high-concentration tungstate clusters. The number of tungstate clusters per 10×10$nm^2$ of $ZrO_2$ matrix increases from 0 (W:Zr = 0.5:9) to ~20 (W:Zr = 3 :9).By analyzing Examples 14 to 16 show that when the solvent of system 2 is methanol, ethanol, propanol, or butanol, the tungstate nanocluster-containing composite oxide can be obtained. The nanoclusters have an average particle size of 0.8-0.9 nm, the number of $Na_xWO_y$ nanoclusters is ~21 in the 10×10$nm^2$ region. By analyzing Examples 17 to 20 show that when using different types of alkane metals of precursors A, the tungstate nanocluster-containing composite oxide can be obtained. The nanoclusters have an average particle size of 0.7-1.1 nm, the number of $Na_xWO_y$ nanoclusters is ~3-12 in the 10×10$nm^2$ region. By analyzing Examples 21 to 29 show that when using the synthetical parameters described in claim, the tungstate nanocluster-containing composite oxide can be obtained. The nanoclusters have an average particle size of 0.7-1.4 nm, the number of $Na_xWO_y$ nanoclusters range from 1 to 18 in the 10×10$nm^2$ region. **Examples 30 to 62** present the catalytic results of $OCM_{catalyst}$, cocatalyst and the catalyst composition.

All reaction conditions, parameters, and catalytic performance are shown in table 2. Typically, 0.2 g of catalyst (Q) and 0.1 g of cocatalyst (P) were physically mixed and then loaded in the quartz reactor. $CH_4$, $O_2$, and $N_2$ in a certain ratio were cofed into the reactor through mass flow controllers. The reaction was carried out at ambient pressure and the reaction products were detected by on-line gas chromatography (Fig. 2).

Example 30 shows the catalytic performance of a cocatalyst synthesized by the preparation method described in example 1 mixed with classical $Mn/Na_2WO_4/SiO_2$. The catalytic results of the catalysts tested under the above conditions show that the catalyst composition exhibits very high selectivity and yield of $C_2$.

By analyzing the catalytic performance of examples 31-46 (Table 2), it can be concluded that the promoters prepared when the alkali metal element is Na, K, Li and the promoter element is Zr or Al are compatible with the classical

Mn/Na$_2$WO$_4$/SiO$_2$. The catalyst showed similar catalytic performance to example 17.

By analyzing the catalytic performance of examples 30 and 37 (Table 2), it can be concluded that sodium tungstate can also be used as the tungsten source to synthesize tungstate nanocluster-containing composite oxide. The mixture with as-synthesized composite oxide significantly improves the OCM performance of Mn/Na$_2$WO$_4$/SiO$_2$.

By analyzing the catalytic performances of examples 30 and 38-41 (Table 2), it can be concluded that composite oxides with W:Zr molar ratios of 1:9, 2:9, 3:9, 4:9 and 5:9 are all good cocatalysts to improve the OCM performance of classical Mn/Na$_2$WO$_4$/SiO$_2$. The volcano plot of C$_2$ yield versus W:Zr ratio is similar to that of tungstate nanoclusters concentration versus W:Zr ratio, suggesting a high concentration of tungstate sub-nanometer clusters is beneficial for the catalytic performance with a volcano-type trend.

By analyzing the catalytic performance of example 42 (Table 2) it can be concluded that the NaWZr with a W:Zr ratio of 0.5:9 cannot improve the OCM performance of Mn/Na$_2$WO$_4$/SiO$_2$, likely because the W content thereof is too low to obtain nanoclusters.

By analyzing the catalytic performance of examples 30 and 43-45 (Table 2) it can be concluded that the co-catalyst prepared when using different alcohol solutions is comparable to the classical Mn/Na$_2$WO$_4$/SiO$_2$. The performance of the mixed catalyst is obviously improved.

By analyzing the catalytic performance of examples 46-49 (Table 2), it can be concluded that different performance enhancements are exhibited when different classical OCM catalysts are used in combination with the co-catalyst synthesized by the preparation method described in example 1.

By analyzing the catalytic performance of examples 50-62 (Table 2), it can be concluded that different performance enhancements are exhibited when different classical OCM catalysts are used in combination with the co-catalyst synthesized by the preparation method described in example 17-29.

Table 1-1

| Parameters | Example1 | Example2 | Example3 | Example4 | Example5 | Example6 | Example7 | Example8 |
|---|---|---|---|---|---|---|---|---|
| Alkali metal species of A | Na | Na | K | K | Na, K | Li | Na | Na |
| Precursor of A | NaOH | NaOH | KOH | KOH | NaOH, KOH | LiOH | NaOH | NaOH |
| Atom % in A | 40 | 40 | 40 | 40 | 40 | 35 | 40 | 40 |
| Additive element of M | Zr | Al | Zr | Al | Zr | Zr | Zr, Al | Zr |
| Precursor of M | $Zr(n\text{-}BuO)_4$ | $Al(i\text{-}PrO)_3$ | $Zr(n\text{-}BuO)_4$ | $Al(i\text{-}PrO)_3$ | $Zr(n\text{-}BuO)_4$ | $Zr(n\text{-}BuO)_4$ | $Zr(n\text{-}BuO)_4$, $Al(i\text{-}PrO)_3$ | $Zr(n\text{-}BuO)_4$ |
| Atom % inM | 45 | 45 | 45 | 45 | 45 | 48.8 | 45 | 45 |
| Precursor of tungsten | $WCl_6$ | $WCl_6$ | $WCl_6$ | $WCl_6$ | $WCl_6$ | $WCl_6$ | $WCl_6$ | $Na_2WO_4$ |
| Atom % of tungsten | 15 | 15 | 15 | 15 | 15 | 16.2 | 15 | 15 |
| Mole ration of A/tungsten | 2.7 | 2.7 | 2.7 | 2.7 | 2.7 | 2.2 | 2.7 | 2.7 |
| Mole ration of tungsten/M | 3: 9 | 3: 9 | 3: 9 | 3: 9 | 3: 9 | 3: 9 | 3: 9 | 3: 9 |
| Type of alcohol in System 2 | ethanol | ethanol | ethanol | ethanol | ethanol | ethanol | ethanol | ethanol |
| Dip time (min) | 10 | 10 | 10 | 10 | 10 | 10 | 10 | 10 |
| Number of $Na_xWO_y$ nanoclusters (per $100\,nm^2$) | 21 | 21 | 21 | 21 | 21 | 21 | 21 | 21 |
| Size of $Na_xWO_y$ nanoclusters (nm) | 0.8 | 0.9 | 0.9 | 0.9 | 0.9 | 0.9 | 0.9 | 0.9 |
| Specific surface area ($m^2/g$) | 0.7 | 0.8 | 0.7 | 0.8 | 1.0 | 0.9 | 0.7 | 0.5 |

Table 1-2

| Parameters | Example 9 | Example 10 | Example11 | Example12 | Example 13 | Example14 | Example15 | Example16 |
|---|---|---|---|---|---|---|---|---|
| Alkali metal species of A | Na | Na | Na | Na | Na | Na | Na | Na |
| Precursor of A | NaOH | NaOH | NaOH | NaOH | NaOH | NaOH | NaOH | NaOH |
| Atom % in A | 50 | 45 | 35 | 30 | 53.5 | 40 | 40 | 40 |
| Additive element of M | Zr | Zr | Zr | Zr | Zr | Zr | Zr | Zr |
| Precursor of M | $Zr(n\text{-}BuO)_4$ | $Zr(n\text{-}BuO)_4$ | $Zr(n\text{-}BuO)_4$ | $Zr(n\text{-}BuO)_4$ | $Zr(n\text{-}BuO)_4$ | $Zr(n\text{-}BuO)_4$ | $Zr(n\text{-}BuO)_4$ | $Zr(n\text{-}BuO)_4$ |
| Atom % in M | 45 | 45 | 45 | 45 | 45 | 45 | 45 | 45 |
| Precursor of tungsten | $WCl_6$ | $WCl_6$ | $WCl_6$ | $WCl_6$ | $WCl_6$ | $WCl_6$ | $WCl_6$ | $WCl_6$ |
| Atom % of tungsten | 5 | 10 | 20 | 25 | 2.5 | 15 | 15 | 15 |
| Mole ration of A/tungsten | 10 | 4.5 | 1.8 | 1.2 | 2.7 | 2.7 | 2.7 | 2.7 |
| Mole ration of tungsten/M | 1:9 | 2:9 | 4:9 | 5:9 | 0.5:9 | 3:9 | 3:9 | 3:9 |
| Type of alcohol in System 2 | ethanol | Ethanol | ethanol | ethanol | ethanol | methanol | n-propanol | n-butanol |
| Dip time (min) | 10 | 10 | 10 | 10 | 10 | 10 | 10 | 10 |
| Number of $Na_xWO_y$ nanoclusters (per 100 nm$^2$) | -3 | -11 | -22 | -22 | -21 | -21 | -21 | -21 |
| Size of $Na_xWO_y$ nanoclusters (nm) | 0.7 | 0.8 | 1.1 | 1.1 | 0.5 | 0.9 | 0.8 | 0.8 |
| Specific surface area (m$^2$/g) | 0.6 | 0.9 | 1.3 | 1.5 | 0.9 | 0.8 | 1.0 | 1.2 |

Table 1-3

| Parameters | Example17 | Example18 | Example19 | Example20 | Example21 | Example22 | Example23 |
|---|---|---|---|---|---|---|---|
| Alkali metal species of A | Mg | Ca | Ba | Sr | Na | Na | Na |
| Precursor of A | $Mg(OH)_2$ | $Ca(OH)_2$ | $Ba(OH)_2$ | $Sr(OH)_2$ | NaOH | $NaHCO_3$ | NaOH, $NaNO_3$ |
| Atom % in A | 10 | 65 | 18 | 2 | 53.5 | 40 | 40 |
| Additive element of M | Si | Ti | La, Ce | Zr, Co | Zr | Zr | Zr |
| Precursor of M | $Si(C_2H_3O_2)_4$ | $Ti(n\text{-}BuO)_4$ | $La(NO_3)_3$, $Sm(NO_3)_3$ | $ZrO(NO_3)_2$, $Co(OAc)_2$ | $Zr(n\text{-}BuO)_4$ | $Zr(n\text{-}BuO)_4$ | $Zr(n\text{-}BuO)_4$ |
| Atom % in M | 81 | 262 | 27 | 90 | 45 | 45 | 45 |
| Precursor of tungsten | $WCl_6$ | $WCl_6$ | $WCl_6$ | $WCl_6$ | $Sr_2WO_4$, $CaWO_4$ | $WCl_6$ | $NH_3WO_3$, $NH_3WO_3 \cdot H_2O$ |
| Atom % of tungsten | 9 | 8.8 | 55 | 8 | 2.5 | 15 | 15 |
| Mole ration of A/tungsten | 1.1 | 7.4 | 0.3 | 0.25 | 2.7 | 2.7 | 2.7 |
| Mole ration of tungsten/M | 1: 9 | 3: 9 | 2: 1 | 1: 11 | 0.5: 9 | 3: 9 | 3: 9 |
| Type of alcohol in System 2 | ethanol | ethanol | ethanol | ethanol | ethanol | methanol, $n$-propanol | ethanol. n-propanol |
| Dip time (min) | 10 | 10 | 10 | 10 | 10 | 10 | 10 |
| Number of $Na_xWO_y$ nanoclusters (per 100 $nm^2$) | ~3 | ~12 | ~3 | ~5 | ~3 | ~18 | ~11 |
| Size of $Na_xWO_y$ nanoclusters (nm) | 0.7 | 0.8 | 1.1 | 0.8 | 1.4 | 1.2 | 1.1 |
| Specific surface area ($m^2/g$) | 0.9 | 0.8 | 1.0 | 1.2 | 1.1 | 0.5 | 0.9 |

Table 1-4

| Parameters | Example18 | Example19 | Example20 | Example21 | Example22 | Example23 |
|---|---|---|---|---|---|---|
| Alkali metal species of A | K | Li, Ca | Ba | Na, Sr | Na | Sr, Ba |
| Precursor of A | $KHCO_3$ | $LiOH$, $Ca(OH)_2$ | $Ba(OH)_2$ | $Na_2CO_3$, Sr $(OH)_2$ | NaOH | $Sr(OH)_2$, $Ba(OH)_2$ |
| Atom % in A | 27 | 40 | 2 | 15 | 20 | 40 |
| Additive element of M | Zr, La | Ce | La, Zr | Zr, Co | Zr | Zr |
| Precursor of M | $ZrOCl_2$, $LaCl_3$ | $CeAc_2$, $CeCl_2$ | $La(NO_3)_3$, $ZrO(NO_3)$ | $ZrO(NO_3)$, $Co(OAc)_2$ | $Zr(n\text{-}BuO)_4$ | citric acid zirconium salt |
| Atom % in M | 22 | 30 | 92 | 70 | 60 | 45 |
| Precursor of tungsten | $WCl_6$ | $NH_4WO_3 \cdot H_2O$ | $WCl_6$ | $WCl_6$ | $Sr_2WO_4$, $CaWO_4$ | $(NH_4)_2WO_4 \cdot 5H_2O$ |
| Atom % of tungsten | 50 | 30 | 6 | 15 | 20 | 15 |
| Mole ration of A/tungsten | 1.2 | 1.3 | 0.3 | 1 | 1 | 2.7 |
| Mole ration of tungsten/M | 1: 23 | 1: 1 | 1: 15 | 1: 4.6 | 3:9 | 3: 9 |
| Type of alcohol in System 2 | $n$-propanol | ethanol | ethanol | ethanol | methanol, ethanol, $n$-propanol | methanol, $n$-propanol |
| Dip time (min) | 10 | 10 | 10 | 10 | 10 | 10 |
| Number of $Na_xWO_y$ nanoclusters (per 100 $nm^2$) | ~11 | ~17 | ~1 | ~5 | ~15 | ~18 |
| Size of $Na_xWO_y$ nanoclusters (nm) | 1.4 | 1.2 | 1.0 | 0.8 | 1.4 | 1.2 |
| Specific surface area ($m^2$/g) | 1.5 | 0.9 | 1.7 | 1.2 | 1.1 | 0.5 |

Table 2-1

| Parameters | Example1 7 | Example1 8 | Example1 9 | Example2 0 | Example2 1 | Example2 2 | Example2 3 | Example2 4 | Example2 5 | Example2 6 |
|---|---|---|---|---|---|---|---|---|---|---|
| Source of catalyst (P) | Example 1 | Example2 | Examples | Example4 | Examples | Example6 | Example7 | Example8 | Example9 | Example1 0 |
| Traditional OCM catalyst (Q) | Mn/ $Na_2WO_4$ /$SiO_2$ | Mn/ $Na_2WO_4$ /$SiO_2$ | Mn/ $Na_2WO_4$ /$SiO_2$ | Mn/ $Na_2WO_4$ /$SiO_2$ | Mn/ $Na_2WO_4$ /$SiO_2$ | Mn/ $Na_2WO_4$ /$SiO_2$ | Mn/ $Na_2WO_4$ /$SiO_2$ | Mn/ $Na_2WO_4$ /$SiO_2$ | Mn/ $Na_2WO_4$ /$SiO_2$ | Mn/ $Na_2WO_4$ /$SiO_2$ |
| Feeding gas ($CH_4$/$O_2$/$N_2$) | 3/1/2.7 | 3/1/2.7 | 3/1/2.7 | 3/1/2.7 | 3/1/2.7 | 3/1/2.7 | 3/1/2.7 | 3/1/2.7 | 3/1/2.7 | 3/1/2.7 |
| Temperatu re (°C) | 750 | 750 | 750 | 750 | 750 | 750 | 750 | 750 | 750 | 750 |
| Selectivity of Q (%) | 48.4 | 48.4 | 48.4 | 48.4 | 48.4 | 48.4 | 48.4 | 48.4 | 48.4 | 48.4 |
| Yield of Q (%) | 8.1 | 8.1 | 8.1 | 8.1 | 8.1 | 8.1 | 8.1 | 8.1 | 8.1 | 8.1 |
| Yield of P (%) | 0.5 | 0.8 | 0.5 | 0.5 | 0.9 | 0.6 | 1.0 | 0.8 | 0.3 | 0.5 |
| Selectivity of mixed catalysts (%) | 73.9 | 70.1 | 67.2 | 65.2 | 75.4 | 63.7 | 77.2 | 65.8 | 77.2 | 72.4 |
| Yield of mixed catalysts (%) | 30.9 | 30.5 | 28.5 | 27.7 | 31.2 | 27.5 | 29.4 | 23.9 | 25.3 | 26.5 |

Table 2-2

| Parameter s | Example2 7 | Example2 8 | Example2 9 | Example3 0 | Example3 1 | Example3 2 | Example 33 | Example 34 | Example 35 | Example 36 |
|---|---|---|---|---|---|---|---|---|---|---|
| Source of catalyst (P) | Example1 1 | Example1 2 | Example 1 3 | Example1 4 | Example1 5 | Example1 6 | Example 1 | Example 1 | Example 1 | Example 1 |
| Traditiona l OCM catalyst (Q) | $Mn/Na_2WO_4/SiO_2$ | $Mn/Na_2WO_4/SiO_2$ | $Mn/Na_2WO_4/SiO_2$ | $Mn/Na_2WO_4/SiO_2$ | $Mn/Na_2WO_4/SiO_2$ | $Mn/Na_2WO_4/SiO_2$ | $La_2O_3$ | $Sm_2O_3$ | Li/MgO | $Ca/CeO_2$ |
| Feeding gas $(CH_4/O_2/ N_2)$ | 3/1/2.7 | 3/1/2.7 | 3/1/2.7 | 3/1/2.7 | 3/1/2.7 | 3/1/2.7 | 3/1/2.7 | 3/1/2.7 | 3/1/2.7 | 3/1/2.7 |
| Temperat ure (°C) | 750 | 750 | 800 | 750 | 750 | 750 | 800 | 800 | 800 | 800 |
| Selectivit y of Q (%) | 48.4 | 48.4 | 60.1 | 48.4 | 48.4 | 48.4 | 47.2 | 33.6 | 45.1 | 52.6 |
| Yield of Q (%) | 8.1 | 8.1 | 23.3 | 8.1 | 8.1 | 8.1 | 16.3 | 9.0 | 11.3 | 8.2 |
| Yield of P (%) | 0.6 | 0.7 | 1.1 | 1.1 | 0.8 | 0.9 | 0.5 | 0.5 | 0.5 | 0.5 |
| Selectivit y of mixed catalysts (%) | 74.2 | 65.9 | 80.1 | 68.9 | 70.1 | 67.7 | 56.1 | 60.0 | 56.5 | 64.7 |
| Yield of mixed catalysts (%) | 29.7 | 27.8 | 20.5 | 24.8 | 28.5 | 28.8 | 20.4 | 16.1 | 16.0 | 12.2 |

Table 2-3

| Parameters | Example50 | Examples 1 | Example52 | Example53 | Example54 | Example55 | Example56 |
|---|---|---|---|---|---|---|---|
| Source of catalyst (P) | Example 17 | Example 18 | Example19 | Example20 | Example21 | Example22 | Example23 |
| Traditional OCM catalyst (Q) | $Mn/Na_2WO_4/SiO_2$ | $Mn/Na_2WO_4/SiO_2$ | $Mn/Na_2WO_4/SiO_2$ | $Mn/Na_2WO_4/SiO_2$ | $Mn/Na_2WO_4/SiO_2$ | $Mn/Na_2WO_4/SiO_2$ | $Mn/Na_2WO_4/SiO_2$ |
| Feeding gas $(CH_4/O_2/N_2)$ | 3/1/2.7 | 3/1/2.7 | 3/1/2.7 | 3/1/2.7 | 3/1/2.7 | 3/1/2.7 | 3/1/2.7 |
| Temperature (°C) | 750 | 750 | 750 | 750 | 750 | 750 | 750 |
| Selectivity of Q (%) | 48.4 | 48.4 | 48.4 | 48.4 | 48.4 | 48.4 | 48.4 |
| Yield of Q (%) | 8.1 | 8.1 | 8.1 | 8.1 | 8.1 | 8.1 | 8.1 |
| Yield of P (%) | 1.1 | 1.2 | 2.0 | 2.1 | 2.2 | 1.9 | 0.3 |
| Selectivity of mixed catalysts (%) | 44.2 | 55.9 | 50.1 | 47.9 | 53.1 | 63.7 | 51.1 |
| Yield of mixed catalysts (%) | 12.5 | 14.2 | 12.8 | 17.6 | 19.4 | 24.2 | 19.5 |

Table 2-4

| Parameters | Example57 | Example58 | Example59 | Example60 | Example61 | Example62 |
|---|---|---|---|---|---|---|
| Source of catalyst (P) | Example24 | Example25 | Example26 | Example27 | Example28 | Example29 |
| Traditional OCM catalyst (Q) | $Mn/Na_2WO_4/SiO_2$ | $Mn/Na_2WO_4/SiO_2$ | $Mn/Na_2WO_4/SiO_2$ | $Mn/Na_2WO_4/SiO_2$ | $Mn/Na_2WO_4/SiO_2$ | $Mn/Na_2WO_4/SiO_2$ |
| Feeding gas ($CH_4/O_2/N_2$) | 3/1/2.7 | 3/1/2.7 | 3/1/2.7 | 3/1/2.7 | 3/1/2.7 | 3/1/2.7 |
| Temperature (°C) | 750 | 750 | 750 | 750 | 750 | 750 |
| Selectivity of Q (%) | 48.4 | 48.4 | 48.4 | 48.4 | 48.4 | 48.4 |
| Yield of Q (%) | 8.1 | 8.1 | 8.1 | 8.1 | 8.1 | 8.1 |
| Yield of P (%) | 1.1 | 0.8 | 0.9 | 0.9 | 0.6 | 1.0 |
| Selectivity of mixed catalysts (%) | 45.8 | 57.5 | 51.7 | 49.5 | 54.7 | 65.3 |
| Yield of mixed catalysts (%) | 13.8 | 15.5 | 14.1 | 18.9 | 20.7 | 25.5 |

**Comparative example 1:**

$WO_3/ZrO_2$ is the comparison example 1. The preparation process of $WO_3/ZrO_2$ is the same as that of example 1, except that no alkali metal Na was added during the synthesis of comparative example 1. The transmission electron microscope result shows that the nanoclusters in the $WO_3/ZrO_2$ catalyst were tungsten oxide instead of sodium tungstate.

Comparative example 1 was tested with $Mn/Na_2WO_4/SiO_2$ in the manner shown in Example 17, with the following results:

The $C_2$ selectivity of $Mn/Na_2WO_4/SiO_2$ was 48.4% and the $C_2$ yield was 8.1%, while the $C_2$ selectivity of $Mn/Na_2WO_4/SiO$ and $WO_3/ZrO_2$ was 46.8% and the $C_2$ yield was 7.6%.From the above results, it can be seen that the comparative example 1 not only failed to improve the selectivity and yield of $Mn/Na_2WO_4/SiO_2$, but also inhibited it. It indicates that the real component of the co-catalyst is not the tungsten oxide nanoclusters, but the tungstate nanoclusters. In the synthesis of the cocatalyst, an alkali metal element is indispensable.

**Comparative example 2:**

The catalyst was synthesized using the same preparation method as in example 1, with the only difference being that the auxiliary element M was not added in the synthesis of comparative example 2. In this case, after the addition of system 1 to system 2, the solution remains clarified, and the corresponding solid catalyst cannot be produced. From this result, it can be seen that the additive element is essential for the synthesis of tungstate nanoclusters.

**Comparative example 3:**

The catalyst was synthesized by the same preparation method as example 1, with the only difference being that the tungsten element was not added in synthesizing comparative example 3. In this case, the resulting comparative example 3 does not contain $Na_xWO_y$ nanoclusters.

Comparative example 3 was tested with $Mn/Na_2WO_4/SiO_2$ in the manner shown in Example 17 with the following results:

The $C_2$ selectivity of $Mn/Na_2WO_4/SiO_2$ was 48.4% and the $C_2$ yield was 8.1%, while the $C_2$ selectivity of $Mn/Na_2WO_4/SiO_2$ with the catalyst composition in comparative example 3 was 45.4% and the $C_2$ yield was 6.8%. From the above results, it can be seen that the comparative example 3 not only failed to improve the selectivity and yield of $Mn/Na_2WO_4/SiO_2$, but also inhibited it. It indicates that elemental tungsten is an indispensable active component of the co-catalyst.

**Comparative example 4:**

The catalyst was synthesized using the same preparation method as in example 1, with the only difference being the replacement of chloride with molybdenum chloride in the synthesis of Comparative example 4, that is the element W in example 1 was changed to the element Mo.

Comparative example 4 was tested with $Mn/Na_2WO_4/SiO_2$ in the manner shown in Example 17 with the following results:

The $C_2$ selectivity of $Mn/Na_2WO_4/SiO_2$ was 48.4% and the $C_2$ yield was 8.1%, while the $C_2$ selectivity of $Mn/Na_2WO_4/SiO_2$ with the catalyst composition in comparative example 4 was 48.2% and the $C_2$ yield was 8.0%. From the above results, it can be seen that the comparative example 4 not only failed to improve the selectivity and yield of $Mn/Na_2WO_4/SiO_2$, but also inhibited it. It indicates that elemental tungsten is an indispensable active component of the co-catalyst.

**Comparative example 5:**

The catalyst was synthesized using the same preparation method as in example 1, with the only difference being that in the synthesis of Comparative example 5, all of system 1 was added to system 2 within 1 min.

The high-resolution electron microscopy images of comparative example 5 are shown in Fig. 17, where it can be seen that the sodium tungstate in comparative example 5 is mainly in the form of large sodium tungstate particles (>10 nm) rather than nanoclusters, indicating that the dropping of acceleration from system 1 into system 2 has an important effect on the structure of the catalyst. The slow addition of system 1 (addition time $\geq$ 2 min) to system 2 allows for the slow hydrolytic crosslinking of the tungsten precursor and the auxiliary element precursor by the alkali metal, thus facilitating the formation of tungstate nanoclusters. Too rapid an addition will result in rapid hydrolysis of the auxiliary element precursors, which will not allow the tungstate to be uniformly dispersed in the nanoclusters.

Comparative example 5 was tested with $Mn/Na_2WO_4/SiO_2$ in the manner shown in Example 17 with the following results:

The $C_2$ selectivity of $Mn/Na_2WO_4/SiO_2$ was 48.4% and the $C_2$ yield was 8.1%, while the $C_2$ selectivity of $Mn/Na_2WO_4/SiO_2$ with the catalyst composition in comparative example 5 was 47.5 % and the $C_2$ yield was 7.6%. From the above results, it can be seen that the comparative example 5 failed to improve the selectivity and yield of $Mn/Na_2WO_4/SiO_2$. It indicates that the tungstate nanocluster is the active structure of the cocatalyst, and the dropping speed of the system 1 can influence the tungstate structure of the cocatalyst species and further influence the reaction performance of the cocatalyst species.

**Comparative example 6:**

The catalyst was synthesized using the same preparation method as in example 1, with the only difference being that the solvent used in the system 2 in the synthesis of comparative example 6 was acetone.

Comparative example 6 was tested with $Mn/Na_2WO_4/SiO_2$ in the manner shown in Example 17 with the following results:

The $C_2$ selectivity of $Mn/Na_2WO_4/SiO_2$ was 48.4% and the $C_2$ yield was 8.1%, while the $C_2$ selectivity of $Mn/Na_2WO_4/SiO_2$ with the catalyst composition in comparative example 6 was 40.3 % and the $C_2$ yield was 6.3%. From the above results, it can be seen that the comparative example 6 failed to improve the selectivity and yield of $Mn/Na_2WO_4/SiO_2$. It indicates that a suitable solvent must be used for the cocatalyst synthesis.

**Comparative example 7:**

The catalyst was synthesized by the same preparation method as in example 1, the only difference is that in the synthesis of Comparative example 7, step 3) was performed by centrifugal washing instead of drying without treatment (i.e., drying the solvent at 40°C for 12 hours in an oven at 80°C). The wt% of tungsten in Proportion 7 was measured by XRF to be less than 0.1%. This indicates that the centrifugal washing step caused the loss of elemental tungsten from the catalyst.

Comparative example 7 was tested with $Mn/Na_2WO_4/SiO_2$ in the manner shown in Example 17 with the following results:

The $C_2$ selectivity of $Mn/Na_2WO_4/SiO_2$ was 48.4% and the $C_2$ yield was 8.1%, while the $C_2$ selectivity of $Mn/Na_2WO_4/SiO_2$ with the catalyst composition in comparative example 7 was 44.2 % and the $C_2$ yield was 6.9%. From the above results, it can be seen that the comparative example 7 failed to improve the selectivity and yield of $Mn/Na_2WO_4/SiO_2$. The above results show that the direct drying of the solvent without treatment as described in step 3) is a very important technical feature of the synthesis method.

**Comparative example 8:**

The catalyst was synthesized using the same preparation method as in example 1, the only difference being that in the synthesis of comparative example 8, the alkali metal precursor used in system 1 was sodium nitrate instead of NaOH. In this case, the pH of system 1 is neutral.

Comparative example 8 was tested with $Mn/Na_2WO_4/SiO_2$ in the manner shown in Example 17 with the following results:

The $C_2$ selectivity of $Mn/Na_2WO_4/SiO_2$ was 48.4% and the $C_2$ yield was 8.1%, while the $C_2$ selectivity of $Mn/Na_2WO_4/SiO_2$ with the catalyst composition in comparative example 8 was 39.5 % and the $C_2$ yield was 5.9%. From the above results, it can be seen that the comparative example 8 failed to improve the selectivity and yield of $Mn/Na_2WO_4/SiO_2$. It indicates that the selection of the alkali metal precursor in the system 1 and the pH value of the system in the synthesis method are very important t technical parameters in the synthesis method.

**Claims**

1.   A composite oxide containing tungstate nanoclusters, **characterized by** comprising an alkali metal element A, a tungsten element W, an auxiliary agent element M, and an oxygen element O; the alkali metal element A, the tungsten element W and the auxiliary agent element M form a compound with the oxygen element O;

the alkali metal element A, the tungsten element W and the auxiliary agent element M form a compound with the oxygen element O refers that the composite oxide is capable of being written in the following formulas: $aAO_x$, $bWO_3$, $cMO_y$, where x, y separately refers the number of oxygen atoms required to satisfy the charge balance of the oxide formed by the alkali metal element A and the auxiliary element M, respectively; a, b, and c separately refers the atomic percent of all non-oxygen elements in the composite oxide; $5\% \leq a \leq 67\%$, $1\% \leq b \leq 60\%$, and $20\% \leq c \leq 94\%$;

the alkali metal element A is one or more selected from the group consisting of Li, Na, K, Mg, Ca, Sr and Ba; the auxiliary agent element M is one or more selected from the group consisting of Si, Zr, Ti, Al, La, Ce and Co; the atomic percentage of the alkali metal element A is 5-67%; the atomic percentage of the tungsten element W is 1-60%; the atomic percent of the auxiliary agent element M is 20-94%; the content of oxygen element in the composite oxide is the sum of the oxygen atom numbers required by the charge balance of the alkali metal element A and the tungsten element W, M;

wherein the atomic percentages of an element in the composite oxide are defined as follows:

Atomic Percentage of an Element

$$= \frac{Total\ Atoms\ Number_{the\ element}}{Total\ Atoms\ Number_{alkali\ A} + Total\ Atoms\ Number_{tungsten\ element\ W} + Total\ Atoms\ Number_{auxiliary\ element\ M}}$$

$\times 100\%$

the tungstate nanocluster is composed of the alkali metal element A, the tungsten element W and the oxygen element O, and the general formula of the tungstate nanocluster is $A_xWO_y$; wherein, $0 < x \leq 2$, y represents the number of oxygen atoms required to satisfy the charge balance of the formula; the tungstate nanoclusters satisfy an enrichment index, which is defined as follows: in any region of $10 \times 10$ $nm^2$, the number of the tungstate nanoclusters is $\geq 3$; preferably, the number of the tungstate nanoclusters is $\geq 5$, more preferably $\geq 10$, and more preferably $\geq 20$;

preferably, the cluster enrichment index detection method comprises: observing the composite oxide under a high-resolution transmission electron microscope, randomly selecting 5 tungstate nanoclusters in a $10 \times 10$ $nm^2$ area, and counting the number of tungstate nanoclusters contained in the region, and taking an average value; preferably, the magnification of the high-resolution transmission electron microscope is 20-500 thousand, preferably 50-400 thousand; preferably, the particle size of the tungstate nanocluster is <10.0 nm, preferably $\leq 5.0$ nm, more preferably $\leq 2.0$mn, and even more preferably $\leq 1.0$ nm; preferably, the specific surface area of the composite oxide is 0.1 to 10.0 $g/m^2$; more preferably 0.5 to 5.0 $g/m^2$; further preferably 1.0 to 2.0 $g/m^2$; preferably, three elements, namely alkali metal element A, tungsten element W and oxygen element O, in the tungstate nanocluster are uniformly distributed, wherein the uniform distribution means that any region in the tungstate nanocluster contains the alkali metal element a, the tungsten element W and the oxygen element O; preferably, the detection method for uniform distribution is as follows: observing the composite oxide under a high-resolution transmission electron microscope, performing component analysis on the tungstate nanoclusters by Energy Dispersive Spectroscopy (EDS), and randomly selecting 10 clusters, wherein any region in each cluster contains an alkali metal element A, a tungsten element W and an oxygen element O; more preferably, the magnification of the high-resolution transmission electron microscope is 20-500 thousand, preferably 50-400 thousand;

preferably, the atomic percentage of the alkali metal element A in the composite oxide is 10% to 65%, and more preferably 15% to 50%; further preferably 10 to 60% by atom, further preferably 20 to 50% by atom, further preferably 30 to 40% by atom; the atomic percentage of the tungsten element W is 2%-55%, more preferably 5%-50%, more preferably 10%-40%, more preferably 20%-30%; the atomic percentage of the auxiliary element M is 22-92%, preferably 25-90%, preferably 30-80%, preferably 40-70%, preferably 50-60%;

preferably, the tungsten element in the tungstate nanocluster exists in the form of a tetracoordinate tungstate, wherein the tetracoordinate means that one tungsten atom has only four oxygen atoms bonded thereto; more preferably, the detection method of the four-coordinate structure is as follows: performing X-ray fine structure spectrum test on the composite oxide, and collecting $L_1$-edge and $L_3$-edge of tungsten element in the X-ray absorption near-edge structure spectroscopy (XANES) and extended X-ray absorption spectroscopy fine structure (EXAFS), by qualitative analysis and data fitting to derive coordination numbers for tungsten atoms;

preferably, after the composite oxide is calcined in the air at 800 °C for 6 hours, the particle size change value $\Delta 1$ of the tungstate nanocluster is $\leq 20\%$, and more preferably, $\Delta 1$ is $< 10\%$; the calculation formula is as follows:

$$\Delta 1 = \frac{|Cluster\ Size_{after\ calcination} - Cluster\ Size_{before\ calcination}|}{Cluster\ Size_{before\ calcination}} * 100\%$$

the cluster enrichment index change value $\Delta 2$ of the calcined tungstate nanoclusters is $\leq 20\%$; more preferably, $\Delta 2$ is $\leq 10\%$; the calculation formula is as follows:

$$\Delta 2 = \frac{|Enrichment\ index_{after\ calcination} - Enrichment\ index_{before\ calcination}|}{Enrichment\ index_{before\ calcination}} * 100\%$$

2. The composite oxide according to claim 1, **characterized in that** the alkali metal element A is at least Na, the auxiliary element M is at least Zr or Al, the composite oxide containing tungstate nanoclusters are respectively expressed as NaWZr or NaWAl, the tungstate nanoclusters are composed of alkali metal elements Na, tungsten element W and oxygen element O, and the tungstate nanoclusters have a general formula of $Na_xWO_y$, $0<x\leq2$, y represents the number of oxygen atoms required to satisfy the charge balance of the formula; the atomic percentage of Na in the composite oxide is 5-67%; the atomic percentage of the tungsten element W is 1-60%; the atomic percent of the auxiliary agent element M is 20-94%;

preferably, the atomic percentage of Na in the composite oxide is 10% to 65%, and more preferably 15% to 50%; further preferably 10 to 60% by atom, further preferably 20 to 50% by atom, further preferably 30 to 40% by atom; the atomic percentage of the tungsten element W is 2% -55%, more preferably 5% -50%, more preferably 10% -40%, more preferably 20% -30%; the atomic percentage of the auxiliary element Zr or Al is 22-92%, preferably 25-90%, preferably 30-80%, preferably 40-70%, preferably 50-60%;

in the composite oxide, 0< the molar ratio of Na: W$\leq$5, preferably, 0.1< the molar ratio of Na: W$\leq$4, and more preferably, 0.8$\leq$ the molar ratio of Na: W$\leq$3.5; 1.0$\leq$ the molar ratio of Na: W$\leq$3.0; further preferably, 1.5 $\leq$ the molar ratio of Na: W $\leq$ 2.0; further preferably 1.4 $\leq$ the molar ratio of Na: W $\leq$1.6; the molar ratio of W to Zr in the composite oxide is $\geq$ 0.1; preferably, the molar ratio of W to Zr is 0.2-100; more preferably, 0.23$\leq$ the molar ratio of W: Zr$\leq$10; more preferably, 0.3$\leq$ the molar ratio of W: Zr$\leq$1; further preferably, 0.4$\leq$ the molar ratio of W: Zr$\leq$0.5.

3. A method for preparing a composite oxide containing tungstate nanoclusters of claim 1 or 2, **characterized by** comprising the following steps:

1) respectively preparing a solution system 1 and a solution system 2, wherein the solution system 1 and the solution system 2 are both transparent solutions; the transparent solutions are solutions without obvious suspended matters; the solutions are not layered, and when light penetrates through the solutions, the Tyndall effect is not generated;

2) adding the solution system 1 into the stirred solution system 2 within 2-200 minutes until a turbid liquid appears, and then continuously stirring the turbid liquid for more than 1 hour, preferably more than 2 hours, and more preferably more than 3 hours; preferably, the dropping time is 2 to 200 minutes, preferably 10 to 100 minutes, and more preferably 20 to 60 minutes; preferably, the stirring is rapid, and the stirring speed is 500-1000 rpm; preferably, the stirring time is 10 minutes to 2 hours, and more preferably 30 minutes to 1 hour;

3) removing the solvent from the product obtained in the step 2) without processing, and drying the obtained solid to obtain a solid product, wherein the non-processing specifically refers to any washing, centrifuging and filtering steps;

preferably, the solvent is removed by drying the solvent, and preferably, the drying is performed by drying the product obtained in the step 2) in an atmosphere with the temperature of 30-50 °C until the solution is volatilized; preferably, the temperature is 40 °C;

preferably, the temperature at which the solid is dried is from 60 to 100 °C, more preferably from 80 to 90 °C; preferably, the drying time is 12 hours or more, more preferably 24 hours or more;

4) annealing the solid product obtained in the step 3) to obtain a composite oxide containing tungstate nanoclusters, preferably, the annealing temperature is 700-900 °C, more preferably 750-850 °C, and more preferably 800 °C; the annealing time is 3 -8 hours, preferably 4-6 hours; the ramp rate of the annealing is 2-10 °C/min, and more preferably 3-5 °C/min;

the preparation method of the solution system 1 comprises the following steps: dissolving an alkali metal element precursor of a compound raw material containing the element in a proper amount of water, and fully stirring to form a transparent solution to obtain solution system 1, wherein the pH of the transparent solution is more than 7, and more preferably $\geq 10$;

the preparation method of the solution system 2 is selected from any one of the following methods: a) mixing and rapidly stirring a tungsten element precursor, an auxiliary agent element precursor and a proper amount of water to form a transparent solution, and obtaining a solution system 2-1; b) mixing and rapidly stirring a tungsten element precursor, an auxiliary element precursor and a proper amount of alcohol to form a transparent solution, and obtaining a solution system 2-2;

the proportion of the alkali metal element precursor, the tungsten element precursor and the auxiliary agent element precursor added into the solution system 1 and the solution system 2 accords with the following formula:

in the solution system 1 and the solution system 2, the atomic percent of a certain element is calculated according to the following formula,

Atomic Percentage of an Element

$$= \frac{Total\ Atoms\ Number_{the\ element}}{Total\ Atoms\ Number_{alkali\ A} + Total\ Atoms\ Number_{tungsten\ element\ W} + Total\ Atoms\ Number_{auxiliary\ element\ M}}$$

$\times 100\%$

the atomic percentage of the alkali metal element A is 5-67%; the atomic percentage of the tungsten element W is 1-60%; the atomic percentage of the auxiliary agent element M is 20-94%;

preferably, the atomic percentage of the alkali metal element A in the composite oxide is 10-65% and more preferably 15-50%; further preferably 10-60%, further preferably 20-50%, further preferably 30-40%; the atomic percentage of the tungsten element W is 2-55%, more preferably 5-50%, more preferably 10-40%, more preferably 20-30%; the atomic percentage of the auxiliary element M is more than 22-92%, preferably 25-90%, preferably 30-80%, preferably 40-70%, preferably 50-60%;

preferably, the concentration of the alkali metal precursor in the solution system 1 is 1-40 wt%, preferably 10-30 wt%, and more preferably 15-25 wt%; preferably, the concentration of the tungsten element precursor in the solution systems 2-1 and 2-2 is 1-30 wt%, preferably 5-25 wt%, and more preferably 10-20 wt%;

preferably, the concentration of the precursor of the auxiliary element in the solution systems 2-1 and 2-2 is 1-50 wt%, preferably 10-40 wt%, and more preferably 20-35 wt%;

preferably, the alkali metal element precursor in the preparation method of the solution system 1 is selected from any one or more of lithium hydroxide, sodium hydroxide, lithium carbonate, sodium bicarbonate, potassium hydroxide, potassium carbonate, potassium bicarbonate, magnesium acetate, calcium hydroxide, calcium acetate, strontium hydroxide, and barium hydroxide;

preferably, the precursor of the auxiliary element in the preparation method of the solution system 2-1 is selected from any one or more of sodium silicate, zirconyl nitrate, zirconium oxychloride, zirconium di(acetate) oxide, zirconyl citrate, titanium nitrate, aluminum nitrate, lanthanum acetate, lanthanum chloride, cerium nitrate, cerium acetate, cerium chloride, cobalt nitrate, and cobalt acetate; the tungsten element precursor is selected from any one or more of sodium tungstate, cesium tungstate, tungsten ethoxide, ammonium tungsten oxide hydrate, strontium tungstate, magnesium tungstate, barium tungstate, ammonium tungstate pentahydrate, ammonium metatungstate hydrate, calcium tungstate, barium tungstate and strontium tungstate;

preferably, the precursor of the tungsten element in the preparation method of the solution system 2-2 is selected from one or two of sodium tungstate and tungsten chloride; the precursor of the auxiliary element is selected from one or more of tetraethyl orthosilicate, zirconium nitrate, zirconium n-butoxide, zirconyl nitrate, zirconium oxychloride, zirconium di(acetate) oxide, zirconium citrate, tetrabutyl titanate, aluminum sec-butoxide, aluminum isopropoxide, lanthanum nitrate, aluminum nitrate, cerium nitrate and cobalt nitrate; the alcoholic solvent is selected from one or more of methanol, ethanol, propanol, and butanol.

4. The method according to claim 3, **characterized in that** the alkali metal element precursor is a sodium element precursor, the auxiliary element precursor is selected from a zirconium element precursor or an aluminum element precursor, the prepared composite oxide containing the tungstate nanocluster is NaWZr or NaWAl, the tungstate nanocluster is composed of an alkali metal element Na, a tungsten element W and an oxygen element O, and the general formula of the tungstate nanocluster is $Na_xWO_y$, $0<x\leq2$, y represents the number of oxygen atoms required to satisfy the charge balance of the formula;

preferably, the alkali metal precursor in the preparation method of the solution system 1 is selected from any one or more of sodium hydroxide, sodium carbonate and sodium bicarbonate;

preferably, the precursor of the auxiliary element in the preparation method of the solution system 2-1 is selected from any one or more of zirconyl nitrate, zirconium oxychloride, zirconium di(acetate) oxide, zirconyl chloride, zirconium citrate and aluminum nitrate; the tungsten element precursor is selected from any one or more of sodium tungstate, cesium tungstate, tungsten ethoxide, ammonium tungsten oxide hydrate, strontium tungstate, magnesium tungstate, barium tungstate, ammonium tungstate pentahydrate, ammonium metatungstate hydrate, calcium tungstate, barium tungstate and strontium tungstate;

preferably, the precursor of the tungsten element in the solution system 2-2 is selected from any one or two of sodium tungstate and tungsten chloride; the precursor of the auxiliary element is selected from any one or more of zirconium nitrate, zirconium n-butoxide, zirconyl nitrate, zirconium oxychloride, zirconium di(acetate) oxide, zirconium citrate, aluminum sec-butoxide, aluminum isopropoxide and aluminum nitrate; the alcoholic solution is selected from one or more of methanol, ethanol, propanol and butanol;

preferably, 0< the molar ratio of Na: W≤mol, preferably, 0.1 of the molar ratio of W methyl alcohol ≤4, more preferably 0.8 step, preferably the molar ratio of alcohol≤ molar ratio; further preferably 1.0 step, preferably the molar ratio of alcohol ≤ molar ratio; more preferably 1.5≤the molar ratio of alcohol≤ molar ratio; further preferably 1.4 step, preferably the molar ratio of alcohol ≤ molar ratio;

the molar ratio of W:Zr is ≥ molar 1; preferably, 0.2≤the molar ratio of W:Zr≤molar ratio; further preferably 0.23≤the molar ratio of W:Zr≤molar; further preferably 0.3 step, preferably the molar ratio of Z≤ molar; further preferably 0.4 step, preferably the molar ratio of Z≤molar ratio;

preferably, the precursor of the auxiliary element in the preparation method of the solution system 2-2 is zirconium n-butoxide, and the precursor of the tungsten element is tungsten chloride; more preferably, the molar ratio of W:Zr≥1:9, preferably≥2:9, and more preferably ≥ 3:9; more preferably, the solution system 1 is NaOH aqueous solution, and the mass percentage of NaOH is 1-60%, preferably 10-50%, more preferably 15-40%;

preferably, the precursor of the auxiliary element in the preparation method of the solution system 2-2 is aluminum isopropoxide, the precursor of the tungsten element is tungsten chloride, and the molar ratio of the tungsten element to the aluminum element is≥1:9, preferably≥2:9, and more preferably≥3:9; more preferably, the solution system 1 is NaOH aqueous solution, and the mass percentage of NaOH is 1-60%, preferably 10-50%, more preferably 15-40%;

preferably, the precursor of the auxiliary element in the preparation method of the solution system 2-2 is zirconium n-butoxide, the precursor of the tungsten element is sodium tungstate, and the molar ratio of W:Zr≥1:9, preferably≥2:9, and more preferably≥3:9; more preferably, the solution system 1 is KOH with a mass percentage of 1-60%, preferably 10-50%, and more preferably 15-40%;

preferably, the precursor of the auxiliary element in the preparation method of the solution system 2-2 is aluminum isopropoxide, the precursor of the tungsten element is sodium tungstate, and the molar ratio of W:Al≥1:9, preferably≥2:9, and more preferably≥3:9; more preferably, the solution system 1 is KOH with a mass percentage of 1-60%, preferably 10-50%, and more preferably 15-40%.

5. A cocatalyst, **characterized in** by comprising the composite oxide containing tungstate nanoclusters of claim 1 or 2;

preferably, the catalyst promoter cannot be used alone as a catalyst for Oxidative Coupling of Methane (OCM) reaction; preferably, the cocatalyst does not have significant OCM activity; preferably, the detection method for the unsignificant OCM activity is as follows: in a bed reactor of co-feeding methane and oxygen, when the catalyst is used as a OCM catalyst and the gas-space-time velocity is$\geq$20000mL/g/h, the bed temperature of a catalyst is$\leq$800 °C, and the reaction pressure is 1atm, the single-pass $C_2$ yield is not higher than 5%; preferably, the yield is quantitatively determined by gas chromatography.

6. A catalyst composition, **characterized by** comprising the composite oxide of claim and at least one $OCM_{catalyst}$ having OCM activity;

preferably, the $OCM_{catalyst}$ having OCM activity means that the catalyst can be used alone for catalyzing OCM reaction; preferably, the meaning of OCM activity is: in a bed reactor of co-feeding methane and oxygen, when the gas-space-time velocity of the catalyst is$\geq$20000mL/g/h, the bed temperature of the catalyst is$\leq$800 °C, and the reaction pressure is 1 atm, the catalyst is used as an OCM catalyst and have a single pass $C_2$ yield$\geq$5%;
preferably, the $OCM_{catalyst}$ has significant OCM activity, and the meaning thereof is as follows:
in a bed reactor co-fed with methane and oxygen, when the catalyst is used as an OCM catalyst, the gas-space-time velocity is $\geq$ 20000mL/g/h, the catalyst bed temperature is$\leq$800 °C, and the reaction pressure is 1 atm, the catalyst is used as an OCM catalyst and haa a single pass $C_2$ yield$\geq$15%;
preferably, the mass ratio of the $OCM_{catalyst}$ to the composite oxide is (0.1-50.0): 1.0; more preferably, the mass ratio is (0.5-20.0): 1.0; more preferably, the mass ratio is (1.0-10.0): 1.0; more preferably, the mass is (2.0-4.0):1.0;
preferably, the yield is quantitatively determined by gas chromatography;
preferably, the $OCM_{catalyst}$ is selected from one or more of tungsten manganese catalyst, rare earth metal oxide, perovskite compound, alkali metal and alkaline earth metal oxide and derivatives of the above catalysts; more preferably tungsten manganese catalyst, lanthanum oxide, samarium oxide, Li/MgO, Ca/CeO$_2$; Preferably, the tungsten manganese catalyst is Mn-Na$_2$WO$_4$/SiO$_2$;
preferably, the distance between the the $OCM_{catalyst}$ and the composite oxide is$\leq$3 mm;
preferably, the catalyst composition is prepared by mixing the composite oxide and $OCM_{catalyst}$ in a physical mixing mode;
preferably, the $OCM_{catalyst}$ is a compound containing a second alkali metal element, a tungsten element, a manganese element, an oxygen element, and a fifth component element; more preferably, the fifth component element is selected from any one or more of Al, Si, Ti, Zr, C, and N, even more preferably, the fifth component element is selected from any one or more of Al, Si, and Ti, and more preferably, the fifth component element is selected from Si; more preferably, the second alkali metal is selected from any one or more of Li, Na and K; more preferably, the second alkali metal is selected from Na;
preferably, the mass percent of the second alkali metal element accounts for the $OCM_{catalyst}$ is 0.1-2.0 wt%; more preferably, the mass percent of the second alkali metal element for the $OCM_{catalyst}$ is 0.5-1.5 wt%;
preferably, the tungsten element accounts for the $OCM_{catalyst}$ is 0.1-5.0 wt%, preferably, the mass percent of the tungsten element accounts for the $OCM_{catalyst}$ is 2.0-4.0 wt%;
preferably, the manganese element accounts for the $OCM_{catalyst}$ is 0.1-10.0 wt%, preferably, the mass percent of the manganese element accounts for the $OCM_{catalyst}$ is 1.0-4.0 wt%.
preferably, the catalyst further comprises an additive; more preferably, the additive is selected from any one or more of heat transfer agent, mass transfer agent, forming agent, wear-resistant enhancing agents, dispersing agents, stabilizing agents and the like.

7. The catalyst composition according to claim 6, **characterized in that** the catalyst composition catalyzes the OCM reaction under the same reaction conditions with selectivity and yield higher than those of the $OCM_{catalyst}$ alone by more than 1.2 times;
preferably, the catalyst composition is capable of realizing $C_2$ selectivity $\geq$ selectively and the single-pass $C_2$ yield $\geq$ pass yield in an OCM bed reactor with co-feeding of methane and oxygen; more preferably, the catalyst composition is capable of achieving $C_2$ selectivity $\geq$selectivity and the single-pass $C_2$ yield $\geq$ 40% in an OCM bed reactor co-fed with methane and oxygen; more preferably, the catalyst composition is capable of achieving $C_2$ selectivity $\geq$ selectivity and the single-pass $C_2$ yield $\geq$ 45% in an OCM bed reactor co-fed with methane and oxygen.

8. The catalyst composition according to claim 7, **characterized in that** the $OCM_{catalyst}$ is Mn/Na$_2$WO$_4$/SiO$_2$, and the composite oxide is NaWZr; preferably, the mass ratio of the $OCM_{catalyst}$ to the composite oxide is 4:1-0.5:1; more preferably 2:1-1:1; preferably, the catalyst composition can realize C2 selectivity $\geq$ 70% and single-pass $C_2$ yield $\geq$ 35% in the OCM bed reactor cofed with methane and oxygen; more preferably, the catalyst composition can realize

$C_2$ selectivity $\geq$ 70% and single-pass $C_2$ yield $\geq$ 40% in the OCM bed reactor cofed with methane and oxygen; more preferably, the catalyst composition can realize $C_2$ selectivity $\geq$ 70% and single-pass $C_2$ yield $\geq$ 45% in the OCM bed reactor cofed with methane and oxygen;

preferably, in an OCM bed reactor with co-feeding of methane and oxygen, the $C_2$ selectivity of the catalyst composition $Mn/Na_2WO_4/SiO_2$-NaWZr can be improved to 1.3 times of $Mn/Na_2WO_4/SiO_2$, the single pass $C_2$ yield is 1.2 times of $Mn/Na_2WO_4/SiO_2$.

9. The catalyst composition according to claim 7, **characterized in that** the $OCM_{catalyst}$ is $La_2O_3$ and the composite oxide NaWZr is the composite oxide of Na, W and Zr, and the mass ratio of the $OCM_{catalyst}$ to the composite oxide is 4:1-1:1; more preferably from 2:1-1:1;

preferably, in an OCM bed reactor with co-feeding of methane and oxygen, the $C_2$ selectivity of the catalyst composition $La_2O_3$-NaWZr can be improved to 1.3 times of $La_2O_3$, the single pass $C_2$ yield is 1.2 times of $La_2O_3$.

10. The catalyst composition according to claim 7, **characterized in that** the $OCM_{catalyst}$ is $Sm_2O_3$ and the composite oxide NaWZr is the composite oxide of Na, W and Zr, and the mass ratio of the $OCM_{catalyst}$ to the composite oxide is 4:1-1:1; more preferably from 2:1-1:1;

preferably, in an OCM bed reactor with co-feeding of methane and oxygen, the $C_2$ selectivity of the catalyst composition $Sm_2O_3$-NaWZr can be improved to 2.0 times of $Sm_2O_3$, the single pass $C_2$ yield is 1.8 times of $Sm_2O_3$.

11. The catalyst composition according to claim 7, **characterized in that** the $OCM_{catalyst}$ is Li/MgO and the composite oxide NaWZr is the composite oxide of Na, W and Zr, and the mass ratio of the $OCM_{catalyst}$ to the composite oxide is 4:1-1:1; more preferably from 2:1-1:1;

preferably, in an OCM bed reactor with co-feeding of methane and oxygen, The $C_2$ selectivity of the catalyst composition Li/MgO-NaWZr can be improved to 2.0 times of Li/MgO. The single pass $C_2$ yield is 1.45 times of Li/MgO.

12. The catalyst composition according to claim 7, **characterized in that** the $OCM_{catalyst}$ is $Ca/CeO_2$ and the composite oxide NaWZr is the composite oxide of Na, W and Zr, and the mass ratio of the $OCM_{catalyst}$ to the composite oxide is 4:1-1:1; more preferably from 2:1-1:1;

preferably, in an OCM bed reactor with co-feeding of methane and oxygen, the $C_2$ selectivity of the catalyst composition $Ca/CeO_2$-NaWZr can be improved to 1.3 times of $Ca/CeO_2$, the single pass $C_2$ yield is 1.5 times of $Ca/CeO_2$.

13. The catalyst composition according to claim 7, **characterized in that** the catalyst composition is $Mn$-$Na_2WO_4/SiO_2$-NaWAl and the $OCM_{catalyst}$ is $Mn$-$Na_2WO_4/SiO_2$ and the composite oxide NaWZr is the composite oxide of Na, W and Zr. The mass ratio of the $OCM_{catalyst}$ to the composite oxide is 4:1-1:1; more preferably from 2:1-1:1;

preferably, the catalyst composition $Mn$-$Na_2WO_4/SiO_2$-NaWAl can realize $C_2$ selectivity $\geq$ 70% and single-pass recovery $C_2$ yield $\geq$ 35% in the OCM bed reactor cofed with methane and oxygen; more preferably, the catalyst composition can realize $C_2$ selectivity $\geq$ 70% and single-pass $C_2$ yield $\geq$ 40% in the OCM bed reactor cofed with methane and oxygen; more preferably, the catalyst composition can realize $C_2$ selectivity $\geq$ 70% and single-pass $C_2$ yield $\geq$ 45% in the OCM bed reactor cofed with methane and oxygen;

preferably, the $C_2$ selectivity of $Mn$-$Na_2WO_4/SiO_2$-NaWAl can be improved to 1.3 times of $Mn$-$Na_2WO_4/SiO_2$, and the single pass $C_2$ yield is 1.2 times of $Mn/Na_2WO_4/SiO_2$.

14. The catalyst composition according to claim 7, **characterized in that** the $OCM_{catalyst}$ is $La_2O_3$, the composite oxide is NaWAl which is a composite oxide of Na, W and Al, and the mass ratio of the $OCM_{catalyst}$ to the composite oxide is 4:1-1:1; more preferably from 2:1 to 1:1;

preferably, the catalyst composition $La_2O_3$-NaWAl in an OCM bed reactor co-feeding with methane and oxygen, the $C_2$ selectivity of $La_2O_3$-NaWAl can be improved to 1.3 times of $La_2O_3$, and the single pass $C_2$ yield is 1.2 times of $La_2O_3$.

15. The catalyst composition according to claim 7, **characterized in that** the $OCM_{catalyst}$ is $Sm_2O_3$, the composite oxide is NaWAl which is a composite oxide of Na, W and Al, and the mass ratio of the $OCM_{catalyst}$ to the composite oxide is 4:1-1:1; more preferably from 2:1 to 1:1;

preferably, the catalyst composition $Sm_2O_3$-NaWAl in an OCM bed reactor co-feeding with methane and oxygen, the $C_2$ selectivity of $Sm_2O_3$-NaWAl can be improved to 1.2 times of $Sm_2O_3$, and the single pass $C_2$ yield is 1.5 times of $Sm_2O_3$.

**16.** The catalyst composition according to claim 7, **characterized in that** the $OCM_{catalyst}$ is Li/MgO, the composite oxide is NaWAl which is a composite oxide of Na, W and Al, and the mass ratio of the $OCM_{catalyst}$ to the composite oxide is 4:1-1:1; more preferably from 2:1 to 1:1;
preferably, the catalyst composition Li/MgO-NaWAl is in an OCM bed reactor with co-feeding of methane and oxygen, and the $C_2$ selectivity of the Li/MgO-NaWAl can be increased to 1.7 times of Li/MgO, and the single-pass $C_2$ yield is 1.8 times of that of Li/MgO.

**17.** The catalyst composition according to claim 7, **characterized in that** the $OCM_{catalyst}$ is $Ca/CeO_2$, the composite oxide is NaWAl which is a composite oxide of Na, W and Al, and the mass ratio of the $OCM_{catalyst}$ to the composite oxide is 4:1-1:1; more preferably from 2:1 to 1:1;
preferably, the catalyst composition $Ca/CeO_2$-NaWAl in an OCM bed reactor co-feeding with methane and oxygen, the $C_2$ selectivity of of NaWAl can be improved to 1.3 times of $Ca/CeO_2$, and the single-pass $C_2$ yield is 1.6 times of $Ca/CeO_2$.

**18.** A use of the catalyst composition of claim 5 and the catalyst composition of any one of claims 6-17 in chemical reactions; preferably, the chemical reaction is a radical conversion reaction; more preferably, the chemical reaction is oxidative coupling of methane. The oxidative coupling of methane refers to a process that carbon-hydrogen bonds of methane are broken under the action of a catalyst, the separated hydrogen and oxygen react to generate water, and carbon-carbon bonds are formed to prepare $C_{2+}$ hydrocarbon.

**19.** The use according to claim 18, **characterized in that** the oxidative coupling of methane takes methane and oxygen as raw material gases, and the reaction is carried out on a bed reactor, and the product comprises $C_2$ hydrocarbons, hydrocarbons and $C_3$ hydrocarbon;

preferably, the feed gas further comprises a diluent gas; more preferably, the diluent gas is selected from at least one of nitrogen, helium, argon;
the ratio of methane to oxygen is 1.0-20.0, preferably 2.0-6.0, more preferably 2.0-5.0;
preferably, the pressure of the raw material gas is 0.1-20.0 MPa, preferably 1.0-8.0 MPa, and more preferably 2.0-6.0 MPa;
preferably, the temperature of the reaction is 600-900 °C, preferably 700-850 °C;
preferably, the space velocity of the reaction is 500-50000 $h^{-1}$, preferably 1000-35000 $h^{-1}$。

**FIG. 1**

Co-feed mode: all reaction gases enter the reactor together and are in contact with the catalyst

One-pass yield: the percentage of the reaction gas converted into the target product by passing through the catalyst bed at one time

**FIG. 2**

**FIG. 3**

**FIG. 4**

FIG. 5

FIG. 6

FIG. 7

FIG. 8

**FIG. 9**

**FIG. 10**

**FIG. 11**

**FIG. 12**

**FIG. 13**

**FIG. 14**

**FIG. 15**

**NaWAl**

WO_x cluster

4 nm

**FIG. 16**

100 nm

**FIG. 17**

FIG. 18

## INTERNATIONAL SEARCH REPORT

International application No.

**PCT/CN2021/124519**

### A. CLASSIFICATION OF SUBJECT MATTER

B01J 23/30(2006.01)i; B01J 23/34(2006.01)i; B01J 35/10(2006.01)i; C07C 2/84(2006.01)i; C07C 9/06(2006.01)i; C07C 11/04(2006.01)i

According to International Patent Classification (IPC) or to both national classification and IPC

### B. FIELDS SEARCHED

Minimum documentation searched (classification system followed by classification symbols)

B01J23,B01J35,C07C2,C07C9,C07C11

Documentation searched other than minimum documentation to the extent that such documents are included in the fields searched

Electronic data base consulted during the international search (name of data base and, where practicable, search terms used)

CNPAT, CNKI, WPI, EPODOC, STN, ISI: 钨酸盐, 钨酸钠, 氧化物, 簇, 纳米, 复合物, 催化, tungstate, oxide, cluster, nano +, complex, composite, catalyst

### C. DOCUMENTS CONSIDERED TO BE RELEVANT

| Category* | Citation of document, with indication, where appropriate, of the relevant passages | Relevant to claim No. |
|---|---|---|
| A | CN 104759291 A (ECO ENVIRONMENTAL ENERGY RESEARCH INSTITUTE LIMITED et al.) 08 July 2015 (2015-07-08)<br>Embodiment 6 | 1-19 |
| A | WO 2013000970 A1 (BAYER INTELLECTUAL PROPERTY GMBH et al.) 03 January 2013 (2013-01-03)<br>embodiment 1 | 1-19 |
| A | CN 102764659 A (HEFEI INSTITUTES OF PHYSICAL SCIENCE, CHINESE ACADEMY OF SCIENCES) 07 November 2012 (2012-11-07)<br>claim 1 | 1-19 |
| A | MOHAMAD, Mohsenmomeni. "Single-step electrochemical anodization for synthesis of hierarchical WO3-TiO2 nanotube arrays on titanium foil as a good photoanode for water splitting with visible light"<br>*JOURNAL OF ELECTROANALYTICAL CHEMISTRY,*<br>Vol. 739, 15 February 2015 (2015-02-15),<br>pages 149-155 | 1-19 |

☐ Further documents are listed in the continuation of Box C.     ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **30 June 2022** | **12 July 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/124519**

| Patent document cited in search report | | | Publication date (day/month/year) | Patent family member(s) | | | Publication date (day/month/year) |
|---|---|---|---|---|---|---|---|
| CN | 104759291 | A | 08 July 2015 | WO | 2015101345 | A1 | 09 July 2015 |
| WO | 2013000970 | A1 | 03 January 2013 | EP | 2540321 | A1 | 02 January 2013 |
| CN | 102764659 | A | 07 November 2012 | | None | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Journal of Molecular Catalysis A: Chemical,* 1999, vol. 145, 1-44
- *Nanoscale,* 2021, vol. 13, 6283
- *Nature Chemistry,* 2009, vol. 1, 722-728
- *ACS Catal.,* 2017, vol. 7, 2181-2198
- *J. Catal.,* 2004, vol. 227, 479-491
- *Topics in Catalysis,* 1998, vol. 6, 87-99
- *Advances in Chemical Engineering and Science,* 2014, vol. 4, 250-257
- *J. Phys. Chem. C,* 2008, vol. 112, 6869-6879
- Journal of Molecular Catalysis (China). Shuben Li group of Lanzhou Institute of Chemical Physics, 1992, vol. 6, 427-433
- *ACS Catalysis,* 2019, vol. 9, 5912-5928
- *Applied Catalysis A: General,* 2012, vol. 425, 53-61
- *Applied Catalysis A: General,* 2002, vol. 225, 271-284
- *Ind. Eng. Chem. Res.,* 2006, vol. 45, 7077
- *Energy Conversion and Management,* 2019, vol. 198, 111789
- *Chinese Journal of Catalysis,* 2021, vol. 42, 1117-1125
- *J. Am. Chem. Soc.,* 1987, vol. 109, 7900-7901
- *J. Mol. Catal. A: Chem.,* 2017, vol. 426, 326-342
- *ACS Catal.,* 2016, vol. 6, 4340
- **ARUTYUNOV et al.** *J. Mol. Catal. A: Chem.,* 2017, vol. 426, 326
- *ChemCαtChem,* 2011, vol. 3, 1935
- *Appl. Catal.,* vol. A 2012, 425
- **BECK, B. et al.** *Catal. Today,* 2014, vol. 228, 212
- **ARUTYUNOV, V. et al.** *J. Mol. Catal. A: Chem.,* 2017, vol. 426, 326
- *Journal of Molecular Catalysis (China),* 1992, vol. 6, 427-433